(19) Europäisches Patentamt
European Patent Office
Office européen des brevets

(11) EP 3 929 180 B1

(12) # EUROPEAN PATENT SPECIFICATION

(45) Date of publication and mention of the grant of the patent:
**04.03.2026 Bulletin 2026/10**

(21) Application number: **20783641.2**

(22) Date of filing: **24.03.2020**

(51) International Patent Classification (IPC):
***C07D 215/56*** (2006.01) ***A01N 43/42*** (2006.01)
***A01N 43/84*** (2006.01) ***A01P 1/00*** (2006.01)
***C07D 401/04*** (2006.01) ***C07D 413/04*** (2006.01)
***A01N 43/40*** (2006.01)

(52) Cooperative Patent Classification (CPC):
**C07D 215/56; A01N 43/40; C07D 401/04; C07D 413/04**

(86) International application number:
**PCT/CN2020/080891**

(87) International publication number:
**WO 2020/199981 (08.10.2020 Gazette 2020/41)**

(54) **QUINOLINE CARBOXYLIC ACID ESTER COMPOUND AND PREPARATION METHOD AND USE THEREOF**

CHINOLINCARBONSÄUREESTERVERBINDUNG UND VERFAHREN ZU IHRER HERSTELLUNG SOWIE IHRE VERWENDUNG

COMPOSÉ ESTER D'ACIDE QUINOLÉINE CARBOXYLIQUE ET SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.04.2019 CN 201910273279**
**13.06.2019 CN 201910512103**

(43) Date of publication of application:
**29.12.2021 Bulletin 2021/52**

(73) Proprietor: **Shandong United Pesticide Industry Co., Ltd.**
**Taian City 271033 (CN)**

(72) Inventors:
- **XU, Hui**
  **Shandong 271033 (CN)**
- **TANG, Jianfeng**
  **Shandong 271033 (CN)**
- **CHI, Huiwei**
  **Shandong 271033 (CN)**
- **WU, Jianting**
  **Shandong 271033 (CN)**
- **HAN, Jun**
  **Shandong 271033 (CN)**
- **LIU, Ying**
  **Shandong 271033 (CN)**
- **ZHAO, Baoxiu**
  **Shandong 271033 (CN)**
- **ZHANG, Zhenguo**
  **Shandong 271033 (CN)**

(74) Representative: **Gille Hrabal Partnerschaftsgesellschaft mbB**
**Patentanwälte**
**Brucknerstraße 20**
**40593 Düsseldorf (DE)**

(56) References cited:
**EP-A2- 0 203 488** **EP-A2- 0 205 029**
**EP-A2- 0 338 372** **EP-A2- 0 520 277**
**CN-A- 101 289 424** **CN-A- 109 942 488**
**CN-A- 110 066 245** **CN-A- 86 103 954**
**JP-A- S61 277 669** **US-A- 4 563 459**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- JINQING JIANG ET AL: "Development of an Heterologous Immunoassay for Ciprofloxacin Residue in Milk", PHYSICS PROCEDIA, vol. 25, 1 January 2012 (2012-01-01), AMSTERDAM, NL, pages 1829 - 1836, XP055924182, ISSN: 1875-3892, DOI: 10.1016/j.phpro.2012.03.318

## Description

The present application claims priority to Chinese Patent Application No. 201910273279.3 filed before China National Intellectual Property Administration on Apr. 4, 2019 and entitled "QUINOLINE CARBOXYLATE COMPOUND AND PREPARATION METHOD AND USE THEREOF" and Chinese Patent Application No. 201910512103.9 filed before China National Intellectual Property Administration on Jun. 13, 2019 and entitled "QUINOLINE CARBOXYLATE COMPOUND AND PREPARATION METHOD AND USE THEREOF".

TECHNICAL FIELD

The present disclosure relates to the technical field of agricultural bactericides, and in particular to a quinoline carboxylate compound, a composition comprising the same,a preparation method and use thereof, including a method which uses the compound.

BACKGROUND

Bacterial diseases of crop plants are common diseases in agricultural production in China, and are even more harmful than viruses. Bacteria have been a major pathogen second only to fungi. Due to various transmission routes and lack of agents for control as well as long-term successive occurrence, there is increasing difficulty in controlling bacterial diseases. According to incomplete statistics, the current occurrence area of bacterial diseases in China is 120 million mu·times, and the market for control of bacterial diseases has a capacity of over 2 billion yuan. Document EP 0 205 029 A discloses quinoline carboxylates, their preparation methods and parenteral antibacterial drugs.

In the present agricultural production, agents for controlling bacterial diseases mainly include copper preparations (including organic or inorganic copper preparations) that are heavily used, and antibiotic products, wherein, the copper preparations have low efficacy for bacterial control, and many substances containing heavy metals are sprayed into the environment and thus pollute soil, water and food, causing safety concern over the environment and food; on the other hand, the heavy use of antibiotics may cause resistance of pathogenic bacteria in the human body to medical antibiotics. Only a few types of other agents can be used for treating agricultural bacteria, and can only be applied in a small area, limited by resistance and efficacy in practical production. Therefore, there is an urgent need for the development of a novel, safe and green chemical pesticide with low-toxicity and low-residue.

SUMMARY

The invention is set out in the appended set of claims; in particular:
In order to further develop a bactericide with excellent performance, the present disclosure provides a quinoline carboxylate compound as shown in formula (I) below,

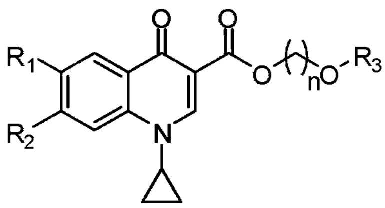

**(I)**

wherein $R_1$ is selected from hydrogen and halogen;

$R_2$ is selected from hydrogen, halogen, $C_1$-$C_6$ alkoxy, halogenated $C_1$-$C_6$ alkoxy, $C_1$-$C_6$ alkylthio, halogenated $C_1$-$C_6$ alkylthio, $C_1$-$C_6$ alkylamino, and di($C_1$-$C_6$ alkyl)amino;

$R_3$ is selected from hydrogen, $C_1$-$C_6$ alkyl and $C_3$-$C_6$ cycloalkyl;

n is an integer from 1 to 4, such as 1, 2, 3 and 4;

the halogen is selected from fluorine, chlorine, bromine and iodine.

Further preferably, in the formula (I),

$R_1$ is selected from fluorine and chlorine;

$R_2$ is selected from hydrogen, fluorine, chlorine, $OCH_3$, $OCH_2CH_3$, $OCH_2CH_2CH_3$, $OCH(CH_3)_2$, $OCH_2CH_2CH_2CH_3$, $OCH_2CH(CH_3)_2$, $OCH(CH_3)(CH_2CH_3)$, $OC(CH_3)_3$, $OCH_2CF_3$, , $SCH_3$, $SCH_2CH_3$, $SCH_2CH_2CH_3$, $SCH(CH_3)_2$, $SCH_2CH_2CH_2CH_3$, $SC(CH_3)_3$, $NHCH_3$, $NHCH_2CH_3$, $N(CH_3)_2$, $N(CH_2CH_3)_2$,;

$R_3$ is selected from H, $CH_3$, $CH_2CH_3$, $CH_2CH_2CH_3$, $CH(CH_3)_2$, $CH_2CH_2CH_2CH_3$, $CH_2CH(CH_3)_2$, $CH(CH_3)(CH_2CH_3)$ and $C(CH_3)_3$;

n is 2 or 3.

As an example, the compound of formula (I) is selected from the following compounds,

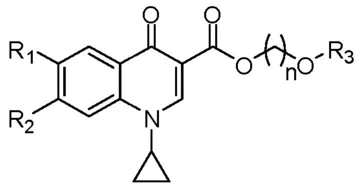

**(I)**

**In** the following table, compounds labelled with a "*" are not according to the current invention. This effect compounds 277-280, 294-297 and 425-436.

| No. | $R_1$ | $R_2$ | $R_3$ | n |
|---|---|---|---|---|
| 1. | F | H | $CH_3$ | 1 |
| 2. | F | H | $CH_2CH_3$ | 1 |
| 3. | F | H | $CH_2CH_2CH_3$ | 1 |
| 4. | F | H | $CH(CH_3)_2$ | 1 |
| 5. | F | H | $CH_2CH_2CH_2CH_3$ | 1 |
| 6. | F | H | $CH_2CH(CH_3)_2$ | 1 |
| 7. | F | H | $CH(CH_3)(CH_2CH_3)$ | 1 |
| 8. | F | H | $C(CH_3)_3$ | 1 |
| 9. | F | H | H | 2 |
| 10. | F | H | $CH_3$ | 2 |
| 11. | F | H | $CH_2CH_3$ | 2 |
| 12. | F | H | $CH_2CH_2CH_3$ | 2 |
| 13. | F | H | $CH(CH_3)_2$ | 2 |
| 14. | F | H | $CH_2CH_2CH_2CH_3$ | 2 |
| 15. | F | H | $CH_2CH(CH_3)_2$ | 2 |
| 16. | F | H | $CH(CH_3)(CH_2CH_3)$ | 2 |
| 17. | F | H | $C(CH_3)_3$ | 2 |
| 18. | F | H | H | 3 |
| 19. | F | H | $CH_3$ | 3 |
| 20. | F | H | $CH_2CH_3$ | 3 |
| 21. | F | H | $CH_2CH_2CH_3$ | 3 |

(continued)

| No. | $R_1$ | $R_2$ | $R_3$ | n |
|---|---|---|---|---|
| 22. | F | H | $CH(CH_3)_2$ | 3 |
| 23. | F | H | $CH_2CH_2CH_2CH_3$ | 3 |
| 24. | F | H | $CH_2CH(CH_3)_2$ | 3 |
| 25. | F | H | $CH(CH_3)(CH_2CH_3)$ | 3 |
| 26. | F | H | $C(CH_3)_3$ | 3 |
| 27. | F | H | 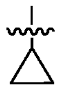 | 3 |
| 28. | F | H | 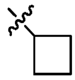 | 3 |
| 29. | F | H | 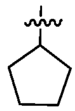 | 3 |
| 30. | F | H | 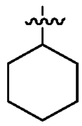 | 3 |
| 31. | F | H | H | 4 |
| 32. | F | H | $CH_3$ | 4 |
| 33. | F | H | $CH_2CH_3$ | 4 |
| 34. | F | H | $CH_2CH_2CH_3$ | 4 |
| 35. | F | H | $CH(CH_3)_2$ | 4 |
| 36. | F | H | $CH_2CH_2CH_2CH_3$ | 4 |
| 37. | F | H | $CH_2CH(CH_3)_2$ | 4 |
| 38. | F | H | $CH(CH_3)(CH_2CH_3)$ | 4 |
| 39. | F | H | $C(CH_3)_3$ | 4 |
| 40. | F | H | 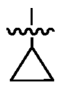 | 4 |
| 41. | F | H | 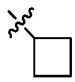 | 4 |
| 42. | F | H | 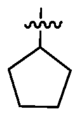 | 4 |
| 43. | F | H | 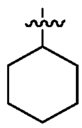 | 4 |
| 44. | Cl | H | H | 2 |
| 45. | Cl | H | $CH_3$ | 2 |
| 46. | Cl | H | $CH_2CH_3$ | 2 |

(continued)

| No. | $R_1$ | $R_2$ | $R_3$ | n |
|---|---|---|---|---|
| 47. | Cl | H | $CH_2CH_2CH_3$ | 2 |
| 48. | Cl | H | $CH(CH_3)_2$ | 2 |
| 49. | Cl | H | $CH_2CH_2CH_2CH_3$ | 2 |
| 50. | Cl | H | $CH_2CH(CH_3)_2$ | 2 |
| 51. | Cl | H | $CH(CH_3)(CH_2CH_3)$ | 2 |
| 52. | Cl | H | $C(CH_3)_3$ | 2 |
| 53. | Cl | H | 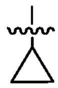 | 2 |
| 54. | Cl | H | 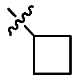 | 2 |
| 55. | Cl | H | 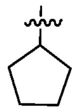 | 2 |
| 56. | Cl | H | 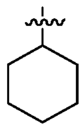 | 2 |
| 57. | Cl | H | H | 3 |
| 58. | Cl | H | $CH_3$ | 3 |
| 59. | Cl | H | $CH_2CH_3$ | 3 |
| 60. | Cl | H | $CH_2CH_2CH_3$ | 3 |
| 61. | Cl | H | $CH(CH_3)_2$ | 3 |
| 62. | Cl | H | $CH_2CH_2CH_2CH_3$ | 3 |
| 63. | Cl | H | $CH_2CH(CH_3)_2$ | 3 |
| 64. | Cl | H | $CH(CH_3)(CH_2CH_3)$ | 3 |
| 65. | Cl | H | $C(CH_3)_3$ | 3 |
| 66. | Cl | H | 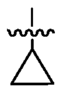 | 3 |
| 67. | Cl | H | 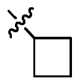 | 3 |
| 68. | Cl | H | 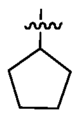 | 3 |
| 69. | Cl | H | 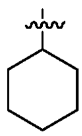 | 3 |
| 70. | Cl | H | H | 4 |
| 71. | Cl | H | $CH_3$ | 4 |

(continued)

| No. | $R_1$ | $R_2$ | $R_3$ | n |
|---|---|---|---|---|
| 72. | Cl | H | $CH_2CH_3$ | 4 |
| 73. | Cl | H | $CH_2CH_2CH_3$ | 4 |
| 74. | Cl | H | $CH(CH_3)_2$ | 4 |
| 75. | Cl | H | $CH_2CH_2CH_2CH_3$ | 4 |
| 76. | Cl | H | $CH_2CH(CH_3)_2$ | 4 |
| 77. | Cl | H | $CH(CH_3)(CH_2CH_3)$ | 4 |
| 78. | Cl | H | $C(CH_3)_3$ | 4 |
| 79. | Cl | H | 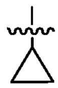 | 4 |
| 80. | Cl | H | 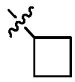 | 4 |
| 81. | Cl | H | 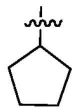 | 4 |
| 82. | Cl | H | 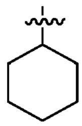 | 4 |
| 83. | Br | H | H | 2 |
| 84. | Br | H | $CH_3$ | 2 |
| 85. | Br | H | $CH_2CH_3$ | 2 |
| 86. | Br | H | $CH_2CH_2CH_3$ | 2 |
| 87. | Br | H | $CH(CH_3)_2$ | 2 |
| 88. | Br | H | $CH_2CH_2CH_2CH_3$ | 2 |
| 89. | Br | H | $CH_2CH(CH_3)_2$ | 2 |
| 90. | Br | H | $CH(CH_3)(CH_2CH_3)$ | 2 |
| 91. | Br | H | $C(CH_3)_3$ | 2 |
| 92. | Br | H | 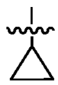 | 2 |
| 93. | Br | H | 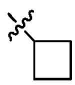 | 2 |
| 94. | Br | H | 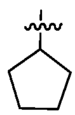 | 2 |
| 95. | Br | H | 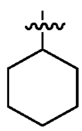 | 2 |
| 96. | Br | H | H | 3 |

(continued)

| No. | $R_1$ | $R_2$ | $R_3$ | n |
|---|---|---|---|---|
| 97. | Br | H | $CH_3$ | 3 |
| 98. | Br | H | $CH_2CH_3$ | 3 |
| 99. | Br | H | $CH_2CH_2CH_3$ | 3 |
| 100. | Br | H | $CH(CH_3)_2$ | 3 |
| 101. | Br | H | $CH_2CH_2CH_2CH_3$ | 3 |
| 102. | Br | H | $CH_2CH(CH_3)_2$ | 3 |
| 103. | Br | H | $CH(CH_3)(CH_2CH_3)$ | 3 |
| 104. | Br | H | $C(CH_3)_3$ | 3 |
| 105. | Br | H | 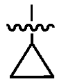 | 3 |
| 106. | Br | H | 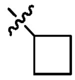 | 3 |
| 107. | Br | H | 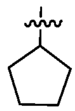 | 3 |
| 108. | Br | H | 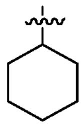 | 3 |
| 109. | Br | H | H | 4 |
| 110. | Br | H | $CH_3$ | 4 |
| 111. | Br | H | $CH_2CH_3$ | 4 |
| 112. | Br | H | $CH_2CH_2CH_3$ | 4 |
| 113. | Br | H | $CH(CH_3)_2$ | 4 |
| 114. | Br | H | $CH_2CH_2CH_2CH_3$ | 4 |
| 115. | Br | H | $CH_2CH(CH_3)_2$ | 4 |
| 116. | Br | H | $CH(CH_3)(CH_2CH_3)$ | 4 |
| 117. | Br | H | $C(CH_3)_3$ | 4 |
| 118. | Br | H | 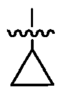 | 4 |
| 119. | Br | H | 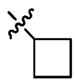 | 4 |
| 120. | Br | H | 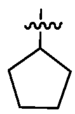 | 4 |
| 121. | Br | H | 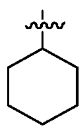 | 4 |

(continued)

| No. | $R_1$ | $R_2$ | $R_3$ | n |
|---|---|---|---|---|
| 122. | I | H | H | 2 |
| 123. | I | H | $CH_3$ | 2 |
| 124. | I | H | $CH_2CH_3$ | 2 |
| 125. | I | H | $CH_2CH_2CH_3$ | 2 |
| 126. | I | H | $CH(CH_3)_2$ | 2 |
| 127. | I | H | $CH_2CH_2CH_2CH_3$ | 2 |
| 128. | I | H | $CH_2CH(CH_3)_2$ | 2 |
| 129. | I | H | $CH(CH_3)(CH_2CH_3)$ | 2 |
| 130. | I | H | $C(CH_3)_3$ | 2 |
| 131. | I | H | H | 3 |
| 132. | I | H | $CH_3$ | 3 |
| 133. | I | H | $CH_2CH_3$ | 3 |
| 134. | I | H | $CH_2CH_2CH_3$ | 3 |
| 135. | I | H | $CH(CH_3)_2$ | 3 |
| 136. | I | H | $CH_2CH_2CH_2CH_3$ | 3 |
| 137. | I | H | $CH_2CH(CH_3)_2$ | 3 |
| 138. | I | H | $CH(CH_3)(CH_2CH_3)$ | 3 |
| 139. | I | H | $C(CH_3)_3$ | 3 |
| 140. | I | H | H | 4 |
| 141. | I | H | $CH_3$ | 4 |
| 142. | I | H | $CH_2CH_3$ | 4 |
| 143. | F | Cl | $CH_3$ | 1 |
| 144. | F | Cl | $CH_2CH_3$ | 1 |
| 145. | F | Cl | $CH_2CH_2CH_3$ | 1 |
| 146. | F | Cl | $CH(CH_3)_2$ | 1 |
| 147. | F | Cl | $CH_2CH_2CH_2CH_3$ | 1 |
| 148. | F | Cl | $CH_2CH(CH_3)_2$ | 1 |
| 149. | F | Cl | $CH(CH_3)(CH_2CH_3)$ | 1 |
| 150. | F | Cl | $C(CH_3)_3$ | 1 |
| 151. | F | Cl | H | 2 |
| 152. | F | Cl | $CH_3$ | 2 |
| 153. | F | Cl | $CH_2CH_3$ | 2 |
| 154. | F | Cl | $CH_2CH_2CH_3$ | 2 |
| 155. | F | Cl | $CH(CH_3)_2$ | 2 |
| 156. | F | Cl | $CH_2CH_2CH_2CH_3$ | 2 |
| 157. | F | Cl | $CH_2CH(CH_3)_2$ | 2 |
| 158. | F | Cl | $CH(CH_3)(CH_2CH_3)$ | 2 |
| 159. | F | Cl | $C(CH_3)_3$ | 2 |

(continued)

| No. | $R_1$ | $R_2$ | $R_3$ | n |
|---|---|---|---|---|
| 160. | F | Cl | 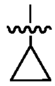 | 2 |
| 161. | F | Cl | 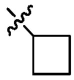 | 2 |
| 162. | F | Cl | 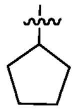 | 2 |
| 163. | F | Cl | 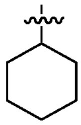 | 2 |
| 164. | F | Cl | H | 3 |
| 165. | F | Cl | $CH_3$ | 3 |
| 166. | F | Cl | $CH_2CH_3$ | 3 |
| 167. | F | Cl | $CH_2CH_2CH_3$ | 3 |
| 168. | F | Cl | $CH(CH_3)_2$ | 3 |
| 169. | F | Cl | $CH_2CH_2CH_2CH_3$ | 3 |
| 170. | F | Cl | $CH_2CH(CH_3)_2$ | 3 |
| 171. | F | Cl | $CH(CH_3)(CH_2CH_3)$ | 3 |
| 172. | F | Cl | $C(CH_3)_3$ | 3 |
| 173. | F | Cl | 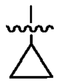 | 3 |
| 174. | F | Cl | 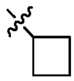 | 3 |
| 175. | F | Cl | 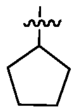 | 3 |
| 176. | F | Cl | 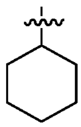 | 3 |
| 177. | F | Cl | H | 4 |
| 178. | F | Cl | $CH_3$ | 4 |
| 179. | F | Cl | $CH_2CH_3$ | 4 |
| 180. | F | Cl | $CH_2CH_2CH_3$ | 4 |
| 181. | F | Cl | $CH(CH_3)_2$ | 4 |
| 182. | F | Cl | $CH_2CH_2CH_2CH_3$ | 4 |
| 183. | F | Cl | $CH_2CH(CH_3)_2$ | 4 |
| 184. | F | Cl | $CH(CH_3)(CH_2CH_3)$ | 4 |

(continued)

| No. | $R_1$ | $R_2$ | $R_3$ | n |
|---|---|---|---|---|
| 185. | F | Cl | $C(CH_3)_3$ | 4 |
| 186. | F | Cl | 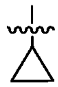 | 4 |
| 187. | F | Cl | 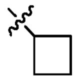 | 4 |
| 188. | F | Cl | 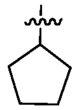 | 4 |
| 189. | F | Cl | 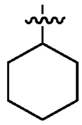 | 4 |
| 190. | H | H | H | 2 |
| 191. | H | H | $CH_3$ | 2 |
| 192. | H | H | H | 3 |
| 193. | H | H | $CH_3$ | 3 |
| 194. | H | F | H | 2 |
| 195. | H | F | $CH_3$ | 2 |
| 196. | H | F | $CH_2CH_3$ | 2 |
| 197. | H | F | $CH_2CH_2CH_3$ | 2 |
| 198. | H | F | $CH(CH_3)_2$ | 2 |
| 199. | H | F | $CH_2CH_2CH_2CH_3$ | 2 |
| 200. | H | F | $CH_2CH(CH_3)_2$ | 2 |
| 201. | H | F | $CH(CH_3)(CH_2CH_3)$ | 2 |
| 202. | H | F | $C(CH_3)_3$ | 2 |
| 203. | H | F | 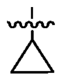 | 2 |
| 204. | H | F | 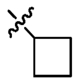 | 2 |
| 205. | H | F | 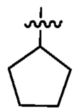 | 2 |
| 206. | H | F | 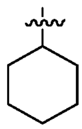 | 2 |
| 207. | H | F | H | 3 |
| 208. | H | F | $CH_3$ | 3 |
| 209. | H | F | $CH_2CH_3$ | 3 |

(continued)

| No. | $R_1$ | $R_2$ | $R_3$ | n |
|---|---|---|---|---|
| 210. | H | F | $CH_2CH_2CH_3$ | 3 |
| 211. | H | F | $CH(CH_3)_2$ | 3 |
| 212. | H | F | $CH_2CH_2CH_2CH_3$ | 3 |
| 213. | H | F | $CH_2CH(CH_3)_2$ | 3 |
| 214. | H | F | $CH(CH_3)(CH_2CH_3)$ | 3 |
| 215. | H | F | $C(CH_3)_3$ | 3 |
| 216. | H | F | 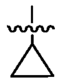 | 3 |
| 217. | H | F | 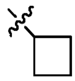 | 3 |
| 218. | H | F | 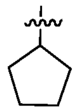 | 3 |
| 219. | H | F | 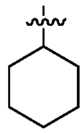 | 3 |
| 220. | H | F | H | 4 |
| 221. | H | F | $CH_3$ | 4 |
| 222. | H | F | $CH_2CH_3$ | 4 |
| 223. | H | F | $CH_2CH_2CH_3$ | 4 |
| 224. | H | F | $CH(CH_3)_2$ | 4 |
| 225. | H | F | $CH_2CH_2CH_2CH_3$ | 4 |
| 226. | H | F | $CH_2CH(CH_3)_2$ | 4 |
| 227. | H | F | $CH(CH_3)(CH_2CH_3)$ | 4 |
| 228. | H | F | $C(CH_3)_3$ | 4 |
| 229. | H | F | 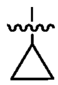 | 4 |
| 230. | H | F | 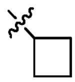 | 4 |
| 231. | H | F | 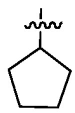 | 4 |
| 232. | H | F | 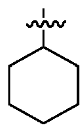 | 4 |
| 233. | H | Cl | H | 2 |
| 234. | H | Cl | $CH_3$ | 2 |

(continued)

| No. | $R_1$ | $R_2$ | $R_3$ | n |
|---|---|---|---|---|
| 235. | H | Cl | $CH_2CH_3$ | 2 |
| 236. | H | Cl | $CH_2CH_2CH_3$ | 2 |
| 237. | H | Cl | $CH(CH_3)_2$ | 2 |
| 238. | H | Cl | $CH_2CH_2CH_2CH_3$ | 2 |
| 239. | H | Cl | $CH_2CH(CH_3)_2$ | 2 |
| 240. | H | Cl | $CH(CH_3)(CH_2CH_3)$ | 2 |
| 241. | H | Cl | $C(CH_3)_3$ | 2 |
| 242. | H | Cl | 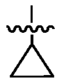 | 2 |
| 243. | H | Cl | 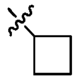 | 2 |
| 244. | H | Cl | 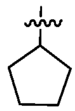 | 2 |
| 245. | H | Cl | 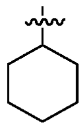 | 2 |
| 246. | H | Cl | H | 3 |
| 247. | H | Cl | $CH_3$ | 3 |
| 248. | H | Cl | $CH_2CH_3$ | 3 |
| 249. | H | Cl | $CH_2CH_2CH_3$ | 3 |
| 250. | H | Cl | $CH(CH_3)_2$ | 3 |
| 251. | H | Cl | $CH_2CH_2CH_2CH_3$ | 3 |
| 252. | H | Cl | $CH_2CH(CH_3)_2$ | 3 |
| 253. | H | Cl | $CH(CH_3)(CH_2CH_3)$ | 3 |
| 254. | H | Cl | $C(CH_3)_3$ | 3 |
| 255. | H | Cl | H | 4 |
| 256. | H | Cl | $CH_3$ | 4 |
| 257. | H | Cl | $CH_2CH_3$ | 4 |
| 258. | H | Cl | $CH_2CH_2CH_3$ | 4 |
| 259. | H | Cl | $CH(CH_3)_2$ | 4 |
| 260. | H | Cl | $CH_2CH_2CH_2CH_3$ | 4 |
| 261. | H | Cl | $CH_2CH(CH_3)_2$ | 4 |
| 262. | H | Cl | $CH(CH_3)(CH_2CH_3)$ | 4 |
| 263. | H | Cl | $C(CH_3)_3$ | 4 |
| 264. | F | $OCH_3$ | H | 2 |
| 265. | F | $OCH_2CH_3$ | H | 2 |
| 266. | F | $OCH_2CH_2CH_3$ | H | 2 |

(continued)

| No. | $R_1$ | $R_2$ | $R_3$ | n |
|---|---|---|---|---|
| 267. | F | $OCH(CH_3)_2$ | H | 2 |
| 268. | F | $OCH_2CH_2CH_2CH_3$ | H | 2 |
| 269. | F | $OCH_2CH(CH_3)_2$ | H | 2 |
| 270. | F | $OC(CH_3)_3$ | H | 2 |
| 271. | F | $OCF_3$ | H | 2 |
| 272. | F | $OCHF_2$ | H | 2 |
| 273. | F | $OCH_2CH_2Cl$ | H | 2 |
| 274. | F | $OCH_2CHF_2$ | H | 2 |
| 275. | F | $OCH_2CF_3$ | H | 2 |
| 276. | F | $OCH_2CH_2CCl_3$ | H | 2 |
| 277. * | F | 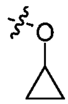 | H | 2 |
| 278. * | F | 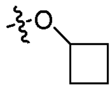 | H | 2 |
| 279. * | F | 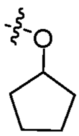 | H | 2 |
| 280. * | F | 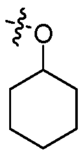 | H | 2 |
| 281. | F | $OCH_3$ | $CH_3$ | 2 |
| 282. | F | $OCH_2CH_3$ | $CH_3$ | 2 |
| 283. | F | $OCH_2CH_2CH_3$ | $CH_3$ | 2 |
| 284. | F | $OCH(CH_3)_2$ | $CH_3$ | 2 |
| 285. | F | $OCH_2CH_2CH_2CH_3$ | $CH_3$ | 2 |
| 286. | F | $OCH_2CH(CH_3)_2$ | $CH_3$ | 2 |
| 287. | F | $OC(CH_3)_3$ | $CH_3$ | 2 |
| 288. | F | $OCF_3$ | $CH_3$ | 2 |
| 289. | F | $OCHF_2$ | $CH_3$ | 2 |
| 290. | F | $OCH_2CH_2Cl$ | $CH_3$ | 2 |
| 291. | F | $OCH_2CHF_2$ | $CH_3$ | 2 |
| 292. | F | $OCH_2CF_3$ | $CH_3$ | 2 |
| 293. | F | $OCH_2CH_2CCl_3$ | $CH_3$ | 2 |
| 294. * | F | 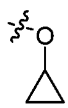 | $CH_3$ | 2 |

(continued)

| No. | $R_1$ | $R_2$ | $R_3$ | n |
|---|---|---|---|---|
| 295. * | F | 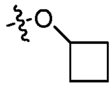 | $CH_3$ | 2 |
| 296. * | F | 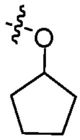 | $CH_3$ | 2 |
| 297. * | F | 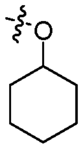 | $CH_3$ | 2 |
| 298. | F | $OCH_2CH_3$ | $CH_2CH_3$ | 2 |
| 299. | F | $OCH_2CH_3$ | $CH_2CH_2CH_3$ | 2 |
| 300. | F | $OCH_2CH_3$ | $CH(CH_3)_2$ | 2 |
| 301. | F | $OCH_2CH_3$ | $CH_2CH_2CH_2CH_3$ | 2 |
| 302. | F | $OCH_2CH_3$ | $CH_2CH(CH_3)_2$ | 2 |
| 303. | F | $OCH_2CH_3$ | $CH(CH_3)(CH_2CH_3)$ | 2 |
| 304. | F | $OCH_2CH_3$ | $C(CH_3)_3$ | 2 |
| 305. | F | $OCH_2CH_3$ | 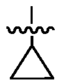 | 2 |
| 306. | F | $OCH_2CH_3$ | 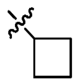 | 2 |
| 307. | F | $OCH_2CH_3$ | 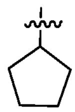 | 2 |
| 308. | F | $OCH_2CH_3$ | 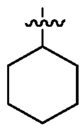 | 2 |
| 309. | F | $OCH_2CH_3$ | H | 3 |
| 310. | F | $OCH_2CH_3$ | H | 4 |
| 311. | F | $OCH_2CH_3$ | $CH_3$ | 1 |
| 312. | F | $OCH_2CH_3$ | $CH_2CH_3$ | 1 |
| 313. | F | $OCH_2CH_3$ | $CH_2CH_2CH_3$ | 1 |
| 314. | F | $OCH_2CH_3$ | $CH_2CH_2CH_2CH_3$ | 1 |
| 315. | F | $OCH_2CH_3$ | $CH_3$ | 3 |
| 316. | F | $OCH_2CH_3$ | $CH_3$ | 4 |
| 317. | F | $OCH_2CH_3$ | $CH_2CH_3$ | 1 |
| 318. | F | $OCH_2CH_3$ | $CH_2CH_3$ | 3 |
| 319. | F | $OCH_2CH_3$ | $CH_2CH_3$ | 4 |

(continued)

| No. | $R_1$ | $R_2$ | $R_3$ | n |
|---|---|---|---|---|
| 320. | F | $OCH_3$ | H | 3 |
| 321. | F | $OCH_3$ | H | 4 |
| 322. | F | $OCH_3$ | $CH_3$ | 1 |
| 323. | F | $OCH_3$ | $CH_3$ | 3 |
| 324. | F | $OCH_3$ | $CH_3$ | 4 |
| 325. | F | $OCH_3$ | $CH_2CH_3$ | 1 |
| 326. | F | $OCH_3$ | $CH_2CH_3$ | 2 |
| 327. | F | $OCH_3$ | $CH_2CH_3$ | 3 |
| 328. | F | $OCH_3$ | $CH_2CH_3$ | 4 |
| 329. | F | $OCH_2CF_3$ | H | 3 |
| 330. | F | $OCH_2CF_3$ | H | 4 |
| 331. | F | $OCH_2CF_3$ | $CH_3$ | 1 |
| 332. | F | $OCH_2CF_3$ | $CH_3$ | 3 |
| 333. | F | $OCH_2CF_3$ | $CH_3$ | 4 |
| 334. | F | $OCH_2CF_3$ | $CH_2CH_3$ | 1 |
| 335. | F | $OCH_2CF_3$ | $CH_2CH_3$ | 2 |
| 336. | F | $OCH_2CF_3$ | $CH_2CH_3$ | 3 |
| 337. | F | $OCH_2CF_3$ | $CH_2CH_3$ | 4 |
| 338. | H | $OCH_2CH_3$ | H | 2 |
| 339. | H | $OCH_2CH_3$ | H | 3 |
| 340. | H | $OCH_2CH_3$ | H | 4 |
| 341. | H | $OCH_2CH_3$ | $CH_3$ | 1 |
| 342. | H | $OCH_2CH_3$ | $CH_3$ | 2 |
| 343. | H | $OCH_2CH_3$ | $CH_3$ | 3 |
| 344. | H | $OCH_2CH_3$ | $CH_3$ | 4 |
| 345. | H | $OCH_2CH_3$ | $CH_2CH_3$ | 1 |
| 346. | H | $OCH_2CH_3$ | $CH_2CH_3$ | 2 |
| 347. | H | $OCH_2CH_3$ | $CH_2CH_3$ | 3 |
| 348. | H | $OCH_2CH_3$ | $CH_2CH_3$ | 4 |
| 349. | H | $OCH_3$ | H | 2 |
| 350. | H | $OCH_3$ | H | 3 |
| 351. | H | $OCH_3$ | H | 4 |
| 352. | H | $OCH_3$ | $CH_3$ | 1 |
| 353. | H | $OCH_3$ | $CH_3$ | 2 |
| 354. | H | $OCH_3$ | $CH_3$ | 3 |
| 355. | H | $OCH_3$ | $CH_3$ | 4 |
| 356. | H | $OCH_3$ | $CH_2CH_3$ | 1 |
| 357. | H | $OCH_3$ | $CH_2CH_3$ | 2 |
| 358. | H | $OCH_3$ | $CH_2CH_3$ | 3 |

(continued)

| No. | $R_1$ | $R_2$ | $R_3$ | n |
|---|---|---|---|---|
| 359. | H | $OCH_3$ | $CH_2CH_3$ | 4 |
| 360. | H | $OCH_2CF_3$ | H | 2 |
| 361. | H | $OCH_2CF_3$ | H | 3 |
| 362. | H | $OCH_2CF_3$ | H | 4 |
| 363. | H | $OCH_2CF_3$ | $CH_3$ | 1 |
| 364. | H | $OCH_2CF_3$ | $CH_3$ | 2 |
| 365. | H | $OCH_2CF_3$ | $CH_3$ | 3 |
| 366. | H | $OCH_2CF_3$ | $CH_3$ | 4 |
| 367. | H | $OCH_2CF_3$ | $CH_2CH_3$ | 1 |
| 368. | H | $OCH_2CF_3$ | $CH_2CH_3$ | 2 |
| 369. | H | $OCH_2CF_3$ | $CH_2CH_3$ | 3 |
| 370. | H | $OCH_2CF_3$ | $CH_2CH_3$ | 4 |
| 371. | F | $SCH_3$ | H | 2 |
| 372. | F | $SCH_2CH_3$ | H | 2 |
| 373. | F | $SCH_2CH_2CH_3$ | H | 2 |
| 374. | F | $SCH(CH_3)_2$ | H | 2 |
| 375. | F | $SCH_2CH_2CH_2CH_3$ | H | 2 |
| 376. | F | $SCH_2CH(CH_3)_2$ | H | 2 |
| 377. | F | $SC(CH_3)_3$ | H | 2 |
| 378. | F | $SCF_3$ | H | 2 |
| 379. | F | $SCH_2CH_2Cl$ | H | 2 |
| 380. | F | $SCH_2CF_3$ | H | 2 |
| 381. | F | $SCH_3$ | $CH_3$ | 2 |
| 382. | F | $SCH_2CH_3$ | $CH_3$ | 2 |
| 383. | F | $SCH_2CH_2CH_3$ | $CH_3$ | 2 |
| 384. | F | $SCH(CH_3)_2$ | $CH_3$ | 2 |
| 385. | F | $SCH_2CH_2CH_2CH_3$ | $CH_3$ | 2 |
| 386. | F | $SCH_2CH(CH_3)_2$ | $CH_3$ | 2 |
| 387. | F | $SC(CH_3)_3$ | $CH_3$ | 2 |
| 388. | F | $SCF_3$ | $CH_3$ | 2 |
| 389. | F | $SCH_2CH_2Cl$ | $CH_3$ | 2 |
| 390. | F | $SCH_2CF_3$ | $CH_3$ | 2 |
| 391. | F | $SCH_2CH_3$ | H | 3 |
| 392. | F | $SCH_2CH_3$ | H | 4 |
| 393. | F | $SCH_2CH_3$ | $CH_3$ | 1 |
| 394. | F | $SCH_2CH_3$ | $CH_3$ | 3 |
| 395. | F | $SCH_2CH_3$ | $CH_3$ | 4 |
| 396. | F | $SCH_2CH_3$ | $CH_2CH_3$ | 1 |
| 397. | F | $SCH_2CH_3$ | $CH_2CH_3$ | 2 |

(continued)

| No. | $R_1$ | $R_2$ | $R_3$ | n |
|---|---|---|---|---|
| 398. | F | $SCH_2CH_3$ | $CH_2CH_3$ | 3 |
| 399. | F | $SCH_2CH_3$ | $CH_2CH_3$ | 4 |
| 400. | F | $NHCH_3$ | H | 2 |
| 401. | F | $NHCH_3$ | $CH_3$ | 2 |
| 402. | F | $NHCH_3$ | $CH_2CH_3$ | 2 |
| 403. | F | $NHCH_3$ | $CH(CH_3)_2$ | 2 |
| 404. | F | $NHCH_3$ | H | 3 |
| 405. | F | $NHCH_3$ | $CH_3$ | 3 |
| 406. | F | $NHCH_3$ | $CH_2CH_3$ | 3 |
| 407. | F | $NHCH_2CH_3$ | H | 2 |
| 408. | F | $NHCH_2CH_3$ | $CH_3$ | 2 |
| 409. | F | $NHCH_2CH_3$ | $CH_2CH_3$ | 2 |
| 410. | F | $NHCH_2CH_3$ | H | 3 |
| 411. | F | $NHCH_2CH_3$ | $CH_3$ | 3 |
| 412. | F | $NHCH_2CH_3$ | $CH_2CH_3$ | 3 |
| 413. | F | $N(CH_3)_2$ | H | 2 |
| 414. | F | $N(CH_3)_2$ | $CH_3$ | 2 |
| 415. | F | $N(CH_3)_2$ | $CH_2CH_3$ | 2 |
| 416. | F | $N(CH_3)_2$ | H | 3 |
| 417. | F | $N(CH_3)_2$ | $CH_3$ | 3 |
| 418. | F | $N(CH_3)_2$ | $CH_2CH_3$ | 3 |
| 419. | F | $N(CH_2CH_3)_2$ | H | 2 |
| 420. | F | $N(CH_2CH_3)_2$ | $CH_3$ | 2 |
| 421. | F | $N(CH_2CH_3)_2$ | $CH_2CH_3$ | 2 |
| 422. | F | $N(CH_2CH_3)_2$ | H | 3 |
| 423. | F | $N(CH_2CH_3)_2$ | $CH_3$ | 3 |
| 424. | F | $N(CH_2CH_3)_2$ | $CH_2CH_3$ | 3 |
| 425. * | F | 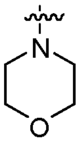 | H | 2 |
| 426. * | F | 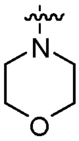 | $CH_3$ | 2 |
| 427. * | F | 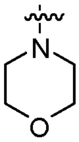 | $CH_2CH_3$ | 2 |

(continued)

| No. | $R_1$ | $R_2$ | $R_3$ | n |
|---|---|---|---|---|
| 428. * | F | 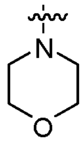 | H | 3 |
| 429. * | F | 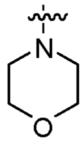 | $CH_3$ | 3 |
| 430. * | F | 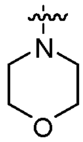 | $CH_2CH_3$ | 3 |
| 431. * | F | 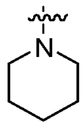 | H | 2 |
| 432. * | F | 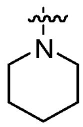 | $CH_3$ | 2 |
| 433. * | F | 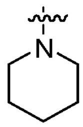 | $CH_2CH_3$ | 2 |
| 434. * | F | 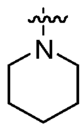 | H | 3 |
| 435. * | F | 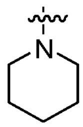 | $CH_3$ | 3 |
| 436. * | F | 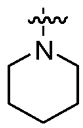 | $CH_2CH_3$ | 3 |

For the sake of reducing the length of the present specification, exemplary groups and/or compounds of the present disclosure are described in the form of the above table. In other words, the above-listed definitions should be understood to include the definitions of the separate groups, and the definitions of the groups in combination.

The present disclosure also provides a preparation method of the compound of formula (I) comprising reacting a compound of formula (II) with a compound of formula (III) to give the compound of formula (I),

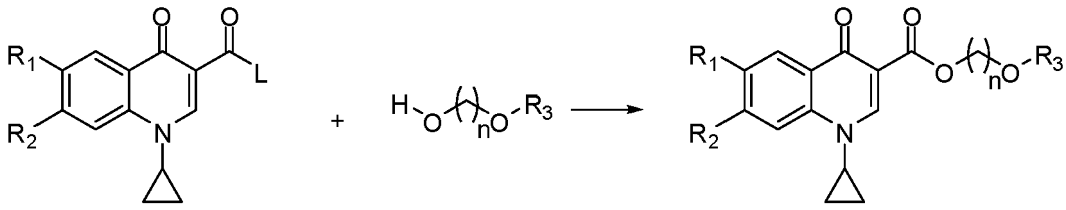

(II) (III) (I)

wherein $R_1$, $R_2$, $R_3$ and n have the definitions as described above; L is selected from a leaving group, for example, a halogen atom, such as fluorine, chlorine, bromine or iodine.

According to an embodiment of the present disclosure, the reaction may be carried out in the presence of a base, wherein the base is selected from an organic base and an inorganic base; and the organic base may be selected from one, two or more of triethylamine, pyridine, etc., and the inorganic base may be selected from one, two or more of sodium carbonate, potassium carbonate, sodium hydroxide, potassium hydroxide, potassium *tert*-butoxide, sodium hydride, etc.

According to an embodiment of the present disclosure, the reaction may be carried out in a solvent, wherein the solvent may be selected from one, two or more of aromatic hydrocarbon solvents, haloalkane solvents, ether solvents, etc., for example, from one, two or more of toluene, dichloromethane, 1,2-dichloroethane, tetrahydrofuran, *tert*-butyl methyl ether, ethyl acetate, etc.

According to an embodiment of the present disclosure, the temperature of the reaction is preferably -10 °C to 50 °C.

Alternatively, the preparation method comprises reacting a compound of formula (V) with a compound of formula (IV) to give the compound of formula (I),

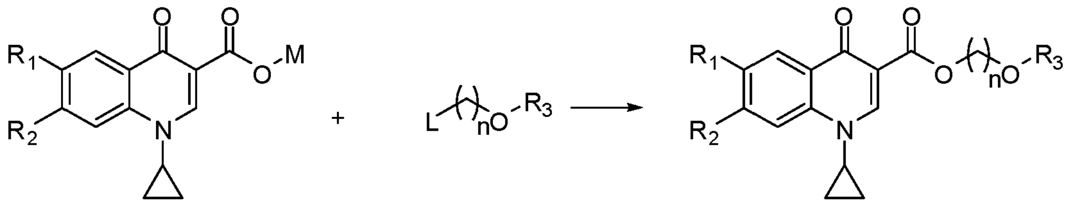

(V) (IV) (I)

wherein $R_1$, $R_2$, $R_3$ and n have the definitions as described above; L is selected from a leaving group, for example, a halogen atom, such as fluorine, chlorine, bromine or iodine; M is selected from an alkali metal, such as sodium or potassium.

According to an embodiment of the present disclosure, the reaction may be carried out in a solvent, for example, a solvent selected from one, two or more of toluene, 1,2-dichloroethane, acetonitrile, butanone, *N,N*-dimethylformamide, dimethylsulfoxide, etc.

According to an embodiment of the present disclosure, the temperature of the reaction is preferably 20 °C to 120 °C.

According to an embodiment of the present disclosure, the compound of formula (II) can be prepared from a compound of formula (II-1),

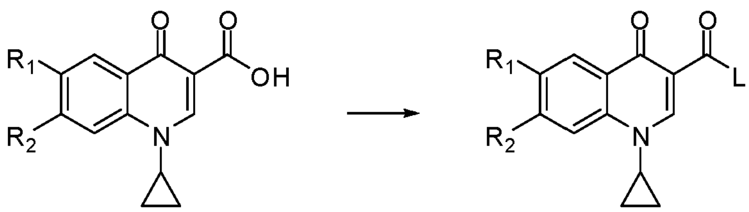

(II-1) (II)

wherein $R_1$ and $R_2$ have the definitions as described above; L is selected from a leaving group, for example, a halogen atom, such as fluorine, chlorine, bromine or iodine.

According to an embodiment of the present disclosure, the halogenating agent may be selected from an acyl halide of an inorganic acid, for example, phosphorus trichloride, phosphorus pentachloride, thionyl chloride, oxalyl chloride, phosphorus oxychloride and phosphorus tribromide.

According to an embodiment of the present disclosure, the halogenation reaction may be carried out in a solvent, wherein the solvent may be selected from one, two or more of aromatic hydrocarbon solvents, halogenated alkane solvents and alkane solvents, for example, from one, two or more of toluene, 1,2-dichloroethane, petroleum ether, etc.

According to an embodiment of the present disclosure, the temperature of the halogenation reaction is preferably 20 °C to 120 °C.

According to an embodiment of the present disclosure, the compound of formula (V) can be prepared from the compound of formula (II-1),

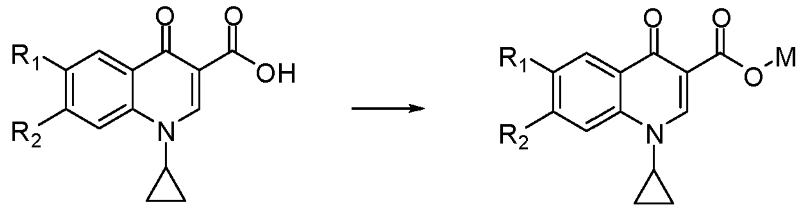

(II-1) (V)

wherein $R_1$ and $R_2$ have the definitions as described above; M is selected from an alkali metal, for example, sodium or potassium.

According to an embodiment of the present disclosure, the reaction may be carried out in the presence of a base, wherein the base may be selected from one or two of inorganic bases, such as sodium carbonate, potassium carbonate, sodium hydroxide, potassium hydroxide, sodium methoxide, sodium ethoxide, potassium tert-butoxide, sodium hydride, etc.

According to the preparation method of the present disclosure, the reaction may be carried out in water and/or an organic solvent, for example, in a solvent selected from one or two of water, methanol, ethanol, tetrahydrofuran, etc.

According to an embodiment of the present disclosure, the temperature of the reaction is preferably 20 °C to 90 °C.

According to an embodiment of the present disclosure, the compound of formula (II-1) is commercially available or can be prepared by using a known method.

According to an embodiment of the present disclosure, where $R_2$ is selected from $C_1$-$C_{16}$ alkoxy, halogenated $C_1$-$C_{16}$ alkoxy, $C_3$-$C_{12}$ cycloalkoxy, $C_1$-$C_{16}$ alkylthio, halogenated $C_1$-$C_{16}$ alkylthio, $C_1$-$C_{16}$ alkylamino, di($C_1$-$C_{16}$ alkyl) amino, and 3-12 membered heterocyclyl, the compound of formula (II-1) can be prepared from a compound of formula (II-2) and a compound of formula (VI),

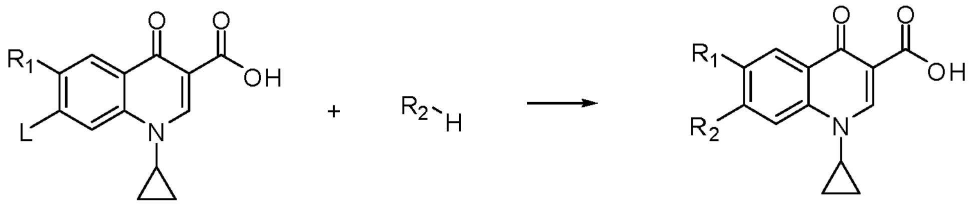

(II-2) (VI) (II-1)

wherein $R_1$ and $R_2$ have the definitions as described above; L is selected from a leaving group, for example, a halogen atom, such as fluorine, chlorine, bromine or iodine.

According to an embodiment of the present disclosure, the reaction may be carried out in the presence or absence of a base, wherein the base is selected from an organic base or an inorganic base; the organic base may be selected from one, two or more of triethylamine, pyridine, etc., and the inorganic base may be selected from one, two or more of sodium carbonate, potassium carbonate, sodium hydroxide, potassium hydroxide, sodium methoxide, sodium ethoxide, potassium tert-butoxide, sodium hydride, sodium, potassium, etc.

According to the preparation method of the present disclosure, the reaction may be carried out in a solvent, wherein the solvent may be selected from one, two or more of aromatic hydrocarbon solvents, amide solvents, sulfone solvents, etc., for example, from one, two or more of toluene, N-methylpyrrolidone, *N,N*-dimethylformamide, *N,N*-dimethylacetamide, dimethylsulfoxide, etc.

According to an embodiment of the present disclosure, the temperature of the reaction is preferably 50 °C to 150 °C.

According to an embodiment of the present disclosure, where $R_2$ is selected from $C_1$-$C_{16}$ alkoxy, halogenated $C_1$-$C_{16}$ alkoxy, $C_1$-$C_{16}$ alkylthio, and halogenated $C_1$-$C_{16}$ alkylthio, the compound of formula (II-1) can also be prepared by

reacting the compound of formula (II-2) with a metal salt (VI-1) of the compound of formula (VI),

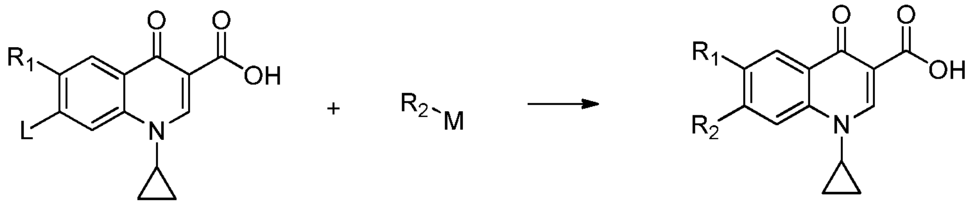

(II-2) (VI-1) (II-1)

wherein $R_1$ and $R_2$ have the definitions as described above; L is selected from a leaving group, for example, a halogen atom, such as fluorine, chlorine, bromine or iodine; M is selected from an alkali metal, for example, sodium or potassium.

According to the preparation method of the present disclosure, the reaction may be carried out in a solvent, wherein the solvent may be selected from one, two or more of aromatic hydrocarbon solvents, amide solvents, sulfone solvents, etc., for example, from one, two or more of toluene, *N*-methylpyrrolidone, *N,N*-dimethylformamide, *N,N*-dimethylacetamide, dimethylsulfoxide, etc.

According to an embodiment of the present disclosure, the temperature of the reaction is preferably 50 °C to 150 °C.

According to an embodiment of the present disclosure, the compound of formula (II-1) can also be commercially available or can be prepared by using a known method:

preparing corresponding benzoyl acetate (IX) by reacting a starting material, substituted acetophenone (X), with dimethyl carbonate or ethyl carbonate, preparing corresponding acrylate (VIII) by reacting the compound (IX) with trialkyl orthoformate, preparing amino acrylate (VII) by reacting the compound (VIII) with cyclopropylamine, preparing quinoline carboxylate (II-3) by cyclizing the compound (VII) under an alkaline condition, and preparing quinoline carboxylate (II-1) by hydrolyzing the compound (II-3),

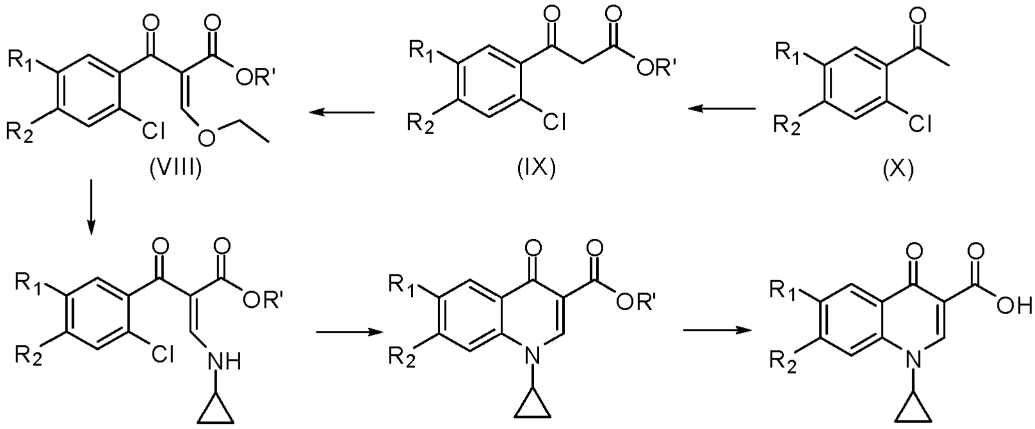

(VIII) (IX) (X) (VII) (II-3) (II-1)

wherein $R_1$ and $R_2$ have the definitions as described above; R' is selected from alkyl, such as methyl or ethyl.

Preferably, the $R_2$ may be selected from hydrogen and halogen, such as hydrogen, fluorine, chlorine, bromine or iodine.

According to an embodiment of the present disclosure, the compound of formula (II-2) can be commercially available or can be prepared by a known method, for example:

preparing corresponding benzoyl acetate (IX-1) by reacting a starting material, substituted acetophenone (X-1), with dimethyl carbonate or ethyl carbonate, preparing corresponding acrylate (VIII-1) by reacting the compound (IX-1) with trialkyl orthoformate, preparing amino acrylate (VII-1) by reacting the compound (VIII-1) with cyclopropylamine, preparing quinoline carboxylate (II-4) by cyclizing the compound (VII-1) under an alkaline condition, and preparing quinoline carboxylate (II-2) by hydrolyzing the compound (II-4),

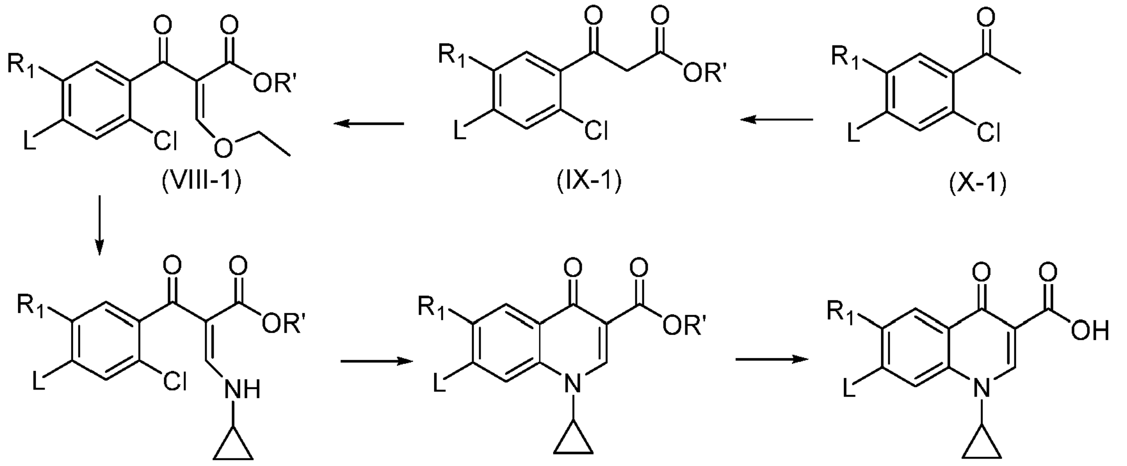

(VIII-1) (IX-1) (X-1)

(VII-1) (II-4) (II-2)

wherein $R_1$ has the definition as described above; L has the definition as described above; R' is selected from alkyl, such as methyl or ethyl.

According to an embodiment of the present disclosure, the reaction can be carried out with reference to a piror literature or a similar method thereof.

According to an embodiment of the present disclosure, the compounds of formulas (X) and (X-1) can also be commercially available or can be prepared by a known method.

In the preparation method of the present disclosure, suitable reaction conditions and starting materials can be selected according to different situations. For example, one substituent can be substituted with another substituent according to the present disclosure in a one-step reaction, or more substituents can be substituted with other substituents according to the present disclosure in the same reaction step.

If the compounds can not be obtained via the above synthetic routes, they can be prepared by deriving other compounds of formula (I) or by conventionally changing the synthetic routes.

The reaction mixture can be post-treated by a conventional method, such as purifying a crude product by mixing with water, phase separation and chromatography, for example, on alumina or silica gel.

An isomer mixture of the compound of formula (I) can be obtained by using the above preparation method. If a pure isomer is desired, the separation can be performed by a conventional method such as crystallization or chromatography.

Unless otherwise indicated, all reactions described above may readily be carried out at atmospheric pressure or the pressure of a particular reaction *per se.*

The present disclosure also provides a use of at least one compound of formula (I) for preparing a bactericide for use in the agricultural field.

The present disclosure also provides a use of at least one compound of formula (I) as a bactericide for use in the agricultural field.

The present disclosure also provides a composition comprising at least one compound of formula (I) as an active ingredient.

The present disclosure also provides a use of the composition as a bactericide for use in the agricultural field.

Preferably, the composition is a bactericidal composition.

According to the present disclosure, the bactericide is preferably a crop bactericide or a plant bactericide.

The present disclosure also provides a method for controlling bacteria (for example, phytopathogens) or diseases caused thereby, comprising applying an effective amount of at least one compound of formula (I) or the composition to a growth medium for the bacteria or diseases.

The examples of bacteria or diseases mentioned below are merely intended to illustrate the present disclosure, but not to confine the scope of the present application.

The compound of formula (I) can be used for controlling the following bacteria or diseases caused thereby:

Gram-negative bacteria: erwinia (causing fire blight in pears, etc.); genus pectobacterium (causing soft rot in cruciferous vegetables, black shank in potatoes, etc.); dikia (causing stem rot in sweet potatoes, bacterial stem rot in maize, bacterial basal rot in rice, black shank in potatoes, rust in pears, etc.); pantoea (causing bacterial wilt in maize, leaf spot by pantoea in maize, bacterial leaf blight in rice beans, canker in drupes, etc.); pseudomonas (causing canker in peaches, bacterial blight in peas, bacterial black spot in crucifer, bacterial leaf spot disease in tomatoes, bacterial spot disease in tomatoes, bacterial black spot in rape, bacterial angular leaf spot in sesame, bacterial angular leaf spot in cucumbers, tobacco

wildfire, bacterial brown spot in maize, bacterial stem blight in broad beans, bacterial spot in soybeans, bacterial spot blight in beet, bacterial pith necrosis in tomatoes, soft rot by pseudomonas aeruginosa in ginseng, etc.); ralstonia (causing various bacterial wilt, etc.); burkholderia (causing bacterial wilt in carnation, onion rot, bacterial spike blight in rice, etc.); acidovorax (causing fruit blotch in melon fruit, brown spot in orchid, brown streak in oat, bacterial leaf spot in konjak, etc.); xanthomonas (causing bacterial blight in rice, bacterial leaf streak in rice, spot disease in peppers and tomatoes, scab disease in peppers and tomatoes, bacterial black spot in mangoes, bacterial leaf spot in peppers, bacterial blight poinsettia, angular leaf spot in cotton, bacterial leaf spot in soybeans, black rot in cruciferae, bacterial blight in cassava, gummosis in sugarcane, bacterial blight in anthurium, bacterial canker in citrus, yellow rot in hyacinth, bacterial shot-hole disease in peaches, angular leaf spot in strawberries, bacterial canker in poplars, etc.); agrobacterium (causing root cancer in rosaceous plants, etc.); xylaria (causing Pierce's disease in grapes, citrus variegated chlorosis, etc.); genus phlobacterium (causing citrus yellow shoot, etc.); enterobacter (causing wilt disease in poplars, etc.); xylophilus (causes bacterial blight in grapes, etc.).

Gram-positive bacteria: clavibacterium (causing ring rot in potatoes, bacterial canker in tomatoes, bacterial wilt in alfalfa, Goss's bacterial wilt and blight in maize, bacterial mosaic in wheat, etc.); streptomyces (mildew) (causing scab disease in potatoes, etc.); curtobacterium (causing bacterial wilt in kidney beans, yellow blister spot in tulips, wilt disease in kidney beans, etc.); arthrobacter (causing leaf blight in winterberries, etc.); rhodococcus (causing fasciation disease in sweet peas, etc.); bacillus (causing bacterial leaf spot by bacillus in maize, white leaf streak in wheat, etc.); rhizoctonia (causing spike blight in cat grass etc.).

Owing to their positive properties, the above-mentioned compounds can advantageously be used for protecting important agricultural and horticultural crops or plants from damage by bacterial germs.

The amount of the compound used to achieve the desired effect will vary depending upon various factors, for example, the compound used, the crop to be protected, the type of the pest, the infection level, the climatic conditions, the route of application and the dosage form employed.

The ingredients of the dosage forms or compositions described herein are selected in accordance with the physical properties of the active ingredient, the route of application and environmental factors such as the soil type, moisture and temperature.

Such dosage forms include liquid agents such as solutions (including emulsifiable concentrates), suspensions and emulsions (including microemulsions and/or suspensions), which may optionally be viscous gels. The dosage forms also include solids such as powders, granules, tablets, pills, films, which may be water-dispersible ("wettable") or water-soluble. The effective ingredient can be microencapsulated and made into suspension or solid dosage form; in addition, the entire dosage form of the active ingredient may also be encapsulated. The capsule can control or delay the release of the effective ingredient. Sprayable dosage forms can be diluted in a suitable medium, and the spray volume used is about one to several hundred liters per hectare. The composition with high concentration is mainly used as an intermediate for further processing.

Typical solid diluents are described in Watkins et al, Handbook of Insecticide Dust Diluents and Carriers, 2nd Ed., Dorland Books, Caldwell, New Jersey. Typical liquid diluents are described in Marsden, SolventsGuide, 2nd Ed., Interscience, New York, 1950. Surfactants and recommended applications are listed in McCutcheon's Detergents and Emulsifiers Annual, Allued Publ. Corp., Ridgewood, New Jersey, and Sisely and Wood, Encyclopedia of Surface Active Agents, Chemical Publishing Co., Inc., New York, 1964. All dosage forms may contain small amounts of additives to reduce foaming, prevent caking, prevent corrosion, prevent microbial growth, etc., or be added with thickeners to increase viscosity.

Surfactants include, for example, polyethoxylated alcohols, polyethoxylated alkylphenols, polyethoxylated sorbitan fatty acid esters, sulfonated dialkyl succinates, alkyl sulfates, alkyl benzene sulfonates, organosilanes, N,N-dialkyl taurates, lignosulfonates, aldehyde condensates for naphthalene sulfonates, polycarboxylates, and polyoxyethylene/polyoxypropylene block copolymers.

Solid diluents include, for example, clays such as bentonite, montmorillonite, attapulgite and kaolin, starches, sugars, silica, talc, celite, urea, calcium carbonate, sodium bicarbonate, sodium sulfate; liquid diluents include, for example, water, N,N-dimethylformamide, dimethylsulfone, N-alkylpyrrolinone, ethylene glycol, polypropylene glycol, paraffin, alkylbenzene, alkylnaphthalene, olive oil, castor oil, linseed oil, tung oil, sesame oil, corn oil, peanut oil, cottonseed oil, soybean oil, rapeseed oil and cacao oil, fatty acid esters, ketones such as cyclohexanone, 2-heptanone, isophorone and 4-hydroxy-4-methyl-2-pentanone, and alcohols such as methanol, cyclohexanol, dodecanol and tetrahydrofuranol.

Solutions, including emulsifiable concentrates, can be prepared by simply mixing the components. Powders and fines may be prepared by mixing or by grinding, usually in a hammer mill or fluid-energy mill. Suspensions are typically prepared by wet milling, for example by the method described in US 3060084. Granules and pills are prepared by spraying the active substance onto freshly prepared granular carriers or by granulation techniques. See Browning, "Agglomeration", Chemical Engineering, December 4, 1967, 147-48; Perry's Chemical Engineer's Handbook, 4th Ed., McGraw-Hill, New York, 1963, 8-57; and WO 9113546. Pills are prepared by the method described in US 4172714, water dispersible and

water soluble granules are prepared by the methods described in US 4144050, US 3920442 and DE 3246493, tablets are prepared by the methods described in US 5180587, US 5232701 and US 5208030. Films may be prepared by the methods described in GB2095558 and US 3299566.

For more information on processing, see US 3235361, column 6, line 16 to column 7, line 19, and Examples 10-41; US 3309192, column 5, line 43 to column 7, line 62, and Examples 8, 12, 15, 39, 41, 52, 53, 58, 132, 138-140, 162-164, 166, 167 and 169-182; US 2891855, column 3, line 66 to column 5, line 17, and Examples 1-4; Klingman, Weed Control as a Science, John Wiley and Sons, Inc., New York 1961, 81-96; and Hance et al., Weed Control Handbook, 8th Ed., Blackwell Scientific Publications, Oxford, 1989.

Herein, for certain applications of the composition, for example, in agriculture, one, two or more of other bactericides, insecticides, acaricides, herbicides, plant growth regulators or fertilizers, etc. can be added to the composition described herein, thereby bringing additional advantages and effects.

Definitions and Description

Unless defined otherwise, all technical and scientific terms used herein have the same meaning as commonly understood by those skilled in the art to which the subject matter of the claims belong. Unless indicated otherwise, all patents, patent applications, and publications referred to herein are incorporated herein by reference in their entirety. If there are multiple definitions for terms herein, those in this section prevail.

In the present specification, groups and substituents thereof can be selected by those skilled in the art to provide stable moieties and compounds. When a substituent is described by a general formula written from left to right, the substituent also includes chemically equivalent substituents that are obtained when the formula is written from right to left. For example, $CH_2O$ is equivalent to $OCH_2$.

When a numerical range defined by only "integer" is recited in the specification and claims of this application, it shall be construed as reciting both endpoints of the range and every integer within the range. For example, an "integer from 1 to 10" should be understood to include every integer of 1, 2, 3, 4, 5, 6, 7, 8, 9 and 10.

The term "halogen" refers to fluorine, chlorine, bromine or iodine.

The term "$C_1$-$C_{16}$ alkyl" refers to a linear or branched saturated monovalent hydrocarbyl group having 1-16 carbon atoms, preferably $C_1$-$C_{10}$ alkyl. "$C_1$-$C_{10}$ alkyl" preferably refers to a linear or branched saturated monovalent hydrocarbyl group having 1, 2, 3, 4, 5, 6, 7, 8, 9 or 10 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or isomers thereof. In particular, the group has 1, 2, 3, 4, 5 or 6 carbon atoms ("$C_1$-$C_6$ alkyl"), and is, for example, methyl, ethyl, propyl, butyl, isopropyl, isobutyl, *sec*-butyl, *tert*-butyl, pentyl or hexyl; more particularly, the group is a linear or branched saturated monovalent hydrocarbyl group having 1, 2, 3 or 4 carbon atoms. The alkyl is, for example, methyl, ethyl, propyl, butyl, isopropyl, isobutyl, *sec*-butyl, *tert*-butyl or isomers thereof.

The above definitions for the term "alkyl", such as "$C_1$-$C_6$ alkyl", are also applicable to other terms containing "$C_1$-$C_6$ alkyl", for example, the terms "$C_1$-$C_6$ alkoxy", "halogenated $C_1$-$C_6$ alkoxy", "$C_1$-$C_6$ alkylthio", "halogenated $C_1$-$C_6$ alkylthio", and "$C_1$-$C_6$ alkylamino".

The term "$C_3$-$C_{12}$ cycloalkyl" refers to a saturated monovalent monocyclic or bicyclic hydrocarbon ring having 3-12 carbon atoms, preferably "$C_3$-$C_{10}$ cycloalkyl". The term "$C_3$-$C_{10}$ cycloalkyl" refers to a saturated monovalent monocyclic or bicyclic hydrocarbon ring having 3, 4, 5, 6, 7, 8, 9 or 10 carbon atoms. The $C_3$-$C_{10}$ cycloalkyl may be a monocyclic hydrocarbyl group such as cyclopropyl, cyclobutyl, cyclopentyl, cyclohexyl, cycloheptyl, cyclooctyl, cyclononyl or cyclodecyl, or a bicyclic hydrocarbyl group such as a decahydronaphthalene ring. In particular, the group has 3, 4, 5 or 6 carbon atoms, and is, for example, cyclopropyl, cyclobutyl, cyclopentyl or cyclohexyl.

The above definitions for the term "cycloalkyl", such as "$C_3$-$C_6$ cycloalkyl", are also applicable to other terms containing "$C_3$-$C_6$ cycloalkyl", for example, the terms "$C_3$-$C_6$ cycloalkoxy", "$C_3$-$C_6$ cycloalkylamino" and "oxo $C_3$-$C_6$ cycloalkylamino".

The term "3-12 membered heterocyclyl" refers to a saturated monovalent monocyclic or bicyclic hydrocarbon ring comprising 1-5 heteroatoms independently selected from N, O and S, and preferably is "3-10 membered heterocyclyl". The term "3-10 membered heterocyclyl" refers to a saturated monovalent monocyclic or bicyclic ring comprising 1-5, preferably 1-3, heteroatoms selected from N, O and S. The heterocyclyl may be connected to the rest of the molecule through any of the carbon atoms or the nitrogen atom (if present). In particular, the heterocyclyl may include, but is not limited to: 4 membered rings such as azetidinyl or oxetanyl; 5 membered rings such as tetrahydrofuranyl, dioxolyl, pyrrolidinyl, imidazolidinyl, pyrazolidinyl or pyrrolinyl; 6 membered rings such as tetrahydropyranyl, piperidinyl, morpholinyl, dithianyl, thiomorpholinyl, piperazinyl or trithianyl; or 7 membered rings such as diazepanyl. More particularly, the heterocyclyl is a 6 membered ring containing at least 1 nitrogen atom, through which the heterocyclyl is connected to the rest of the molecule.

The term "haloalkyl" refers to a linear or branched alkyl group where the hydrogen atoms are partially or fully replaced by halogen atoms, for example, $CF_3CH_2$-. The term "alkoxy" refers to a linear or branched alkyl group that is bonded to the structure through an oxygen atom, for example, $CH_3CH_2O$-.
The term "haloalkoxy" refers to an alkoxy group where the hydrogen atoms of the alkyl group may be partially or fully replaced by halogen atoms, for example, $ClCH_2CH_2O$-.
The term "alkylthio" refers to a linear or branched alkyl group that is bonded to the structure through a sulfur atom, for example, $CH_3CH_2S$-.
The term "haloalkylthio" refers to an alkylthio group where the hydrogen atoms of the alkyl group may be partially or fully replaced by halogen atoms, for example, $ClCH_2CH_2S$-.
The term "alkylamino" refers to a linear or branched alkyl group that is bonded to the structure through a nitrogen atom, for example, $CH_3CH_2NH$-.
The term "di(alkyl)amino" refers to 2 linear or branched alkyl groups that are bonded to the structure through a nitrogen atom, for example, $(CH_3CH_2)_2N$-.

Beneficial Effects

Surprisingly, it is found that the compounds of formula (I) disclosed herein show excellent activity against various bacterial germs in the agricultural field. Moreover, the compounds can achieve an excellent control effect at a very low dosage, and thus can be used for preparing bactericides, particularly bactericides for crops or plants. The compounds are further proved to have good activity for improving the growth and development of crops, to be capable of promoting the height growth of plants, to stimulate the synthesis of chlorophyll and increase the leaf area of the plants, so that the leaves of the crops are greener and thicker, and the photosynthetic efficiency is thus improved, which indirectly improves the immunity of the plants and the capability of resisting the adverse external environment, and makes the plants more robust.
In addition, the compounds of the present disclosure are easy to prepare and high in yield, and thus have good prospects for application.

## DETAILED DESCRIPTION

The technical solutions of the present disclosure will be further illustrated in detail with reference to the following specific examples. The following examples are merely exemplary illustration and explanation of the present disclosure, and should not be construed as confining the protection scope of the present disclosure. All techniques implemented based on the aforementioned contents of the present disclosure are encompassed within the protection scope of the present disclosure.
Unless otherwise specified, the raw materials and reagents used in the following examples are all commercially available products or can be prepared by known methods.
The following methods are used for LC-MS analysis:
Chromatography column: Agilent **ZORBAX SB-C18** 150 mm × 4.6 mm, 5 μm (inner diameter);
Detection wavelength: 254 nm;
Flow rate: 0.8 mL/min;
Column temperature: 30 °C;
Gradient elution conditions:

| Time (min) | Acetonitrile (%) | 0.1% aqueous formic acid (%) |
|---|---|---|
| 0.00 | 50 | 50 |
| 5.00 | 50 | 50 |
| 15.00 | 90 | 10 |
| 20.00 | 90 | 10 |

Synthetic examples

Example 1: methoxymethyl 1-cyclopropyl-6-fluoro-4-oxo-1,4-dihydroquinoline-3-carboxylate (Compound 1)

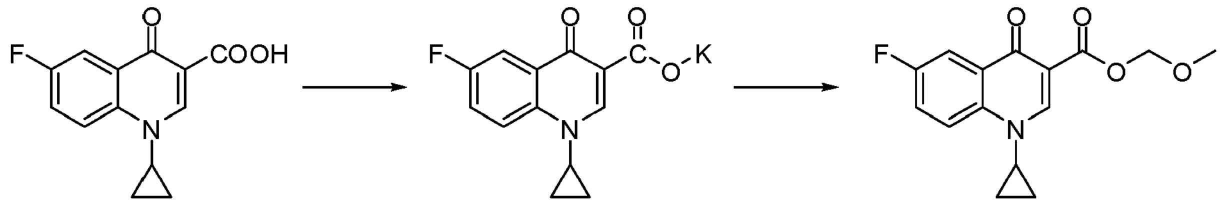

First reaction: potassium 1-cyclopropyl-6-fluoro-4-oxo-1,4-dihydroquinoline-3-carboxylate

1-cyclopropyl-6-fluoro-4-oxo-1,4-dihydroquinoline-3-carboxylic acid (12.3 g, 0.05 mol) and 30% aqueous potassium hydroxide (12 mL) were added to a three-necked flask at room temperature. The reaction was heated to 60 °C for 2 h. Then the heating was stopped, and the water was removed under reduced pressure to give the product (13.6 g, 96% yield).

Second reaction: methoxymethyl 1-cyclopropyl-6-fluoro-4-oxo-1,4-dihydroquinoline-3-carboxylate

Potassium 1-cyclopropyl-6-fluoro-4-oxo-1,4-dihydroquinoline-3-carboxylate (2.85 g, 0.01 mol) and chloromethyl methyl ether (0.96 g, 0.012 mol) were sequentially dissolved in N,N-dimethylformamide (15 mL) at room temperature. The reaction was heated to 100 °C for 6 h. Then the heating was stopped, and the reaction mixture was added with water (15 mL) and extracted with ethyl acetate (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (1.92 g, 66% yield).

LC/MS$[M+H]^+$= 292.1, $[M+Na]^+$ = 314.08, $[M+K]^+$ = 330.05.

Example 2: 2-methoxyethyl 1-cyclopropyl-6-fluoro-4-oxo-1,4-dihydroquinoline-3-carboxylate (Compound 10)

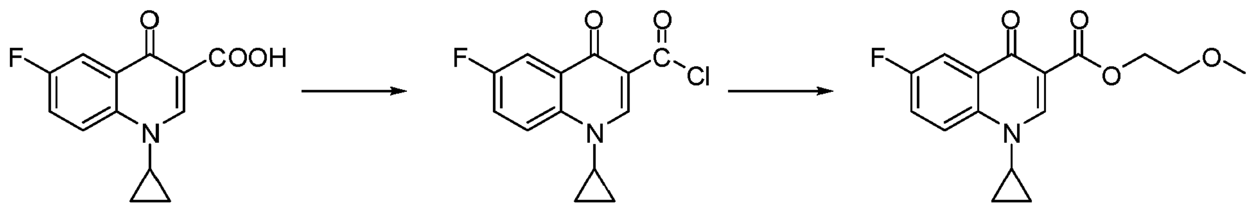

First reaction: 1-cyclopropyl-6-fluoro-4-oxo-1,4-dihydroquinoline-3-carbonyl chloride

1-cyclopropyl-6-fluoro-4-oxo-1,4-dihydroquinoline-3-carboxylic acid (14.9 g, 0.06 mol), dichloroethane (90 mL) and N,N-dimethylformamide (0.2 g) were added to a three-necked flask at room temperature, and the resulting mixture was added dropwise with thionyl chloride (14.6 g, 0.12 mol), and heated to reflux for 5 h. Then the heating was stopped, and the solvent and residual thionyl chloride were removed under reduced pressure to give the product (14.6 g, 92% yield).

Second reaction: 2-methoxyethyl 1-cyclopropyl-6-fluoro-4-oxo-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 1-cyclopropyl-6-fluoro-4-oxo-1,4-dihydroquinoline-3-carbonyl chloride (2.52 g, 0.0095 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (2.63 g, 91% yield).

LC/MS$[M+H]^+$= 306.12, $[M+Na]^+$ = 328.1, $[M+K]^+$ = 344.07.

Example 3: 2-methoxyethyl 6-chloro-1-cyclopropyl-4-oxo-1,4-dihydroquinoline-3-carboxylate (Compound 45)

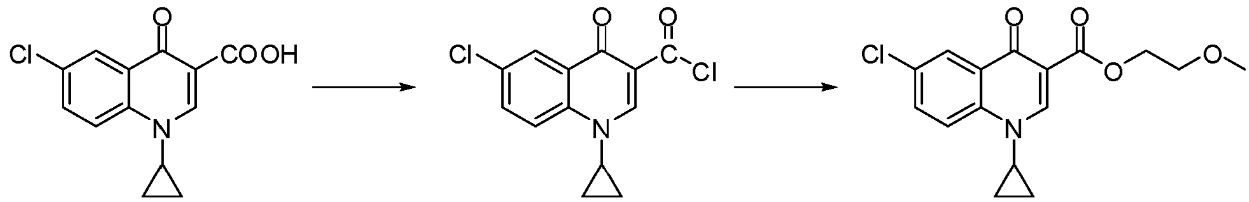

First reaction: 6-chloro-1-cyclopropyl-4-oxo-1,4-dihydroquinoline-3-carbonyl chloride

6-chloro-1-cyclopropyl-4-oxo-1,4-dihydroquinoline-3-carboxylic acid (15.8 g, 0.06 mol), dichloroethane (90 mL) and N,N-dimethylformamide (0.2 g) were added to a three-necked flask at room temperature, and the resulting mixture was added dropwise with hionyl chloride (14.6 g, 0.12 mol), and heated to reflux for 5 h.

Then the heating was stopped, and the solvent and residual thionyl chloride were removed under reduced pressure to give the product (16.2 g, 96% yield).

Second reaction: 2-methoxyethyl 6-chloro-1-cyclopropyl-4-oxo-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 6-chloro-1-cyclopropyl-4-oxo-1,4-dihydroquinoline-3-carbonyl chloride (2.68 g, 0.0095 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (2.81 g, 92% yield).

LC/MS$[M+H]^+$= 322.09, $[M+Na]^+$ = 344.07, $[M+K]^+$ = 360.04.

Example 4: 2-hydroxyethyl-7-chloro-1-cyclopropyl-6-fluoro-4-oxo-1,4-dihydroquinoline-3-carboxylate (Compound 151)

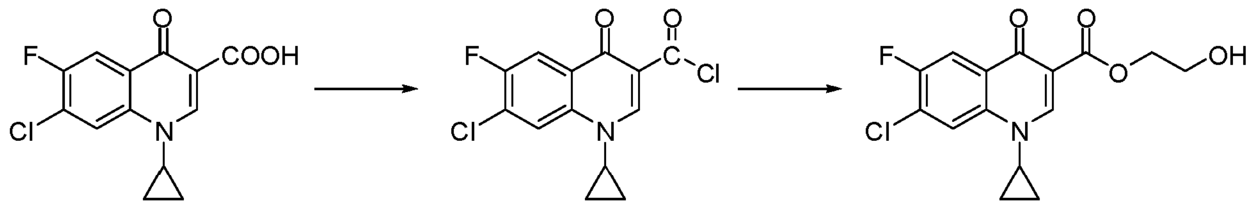

First reaction: 7-chloro-1-cyclopropyl-6-fluoro-4-oxo-1,4-dihydroquinoline-3-carbonyl chloride

7-chloro-1-cyclopropyl-6-fluoro-4-oxo-1,4-dihydroquinoline-3-carboxylic acid (16.9 g, 0.06 mol), dichloroethane (90 mL) and *N,N*-dimethylformamide (0.2 g) were added to a three-necked flask at room temperature, and the resulting mixture was added dropwise with thionyl chloride (14.6 g, 0.12 mol), and heated to reflux for 5 h. Then the heating was stopped, and the solvent and residual thionyl chloride were removed under reduced pressure to give the product (16.2 g, 90% yield).

Second reaction: 2-hydroxyethyl 7-chloro-1-cyclopropyl-6-fluoro-4-oxo-1,4-dihydroquinoline-3-carboxylate

1,2-dihydroxyethane (0.62 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 7-chloro-1-cyclopropyl-6-fluoro-4-oxo-1,4-dihydroquinoline-3-carbonyl

chloride (2.85 g, 0.0095 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (2.32 g, 75% yield).

LC/MS$[M+H]^+$= 326.06, $[M+Na]^+$ = 348.04, $[M+K]^+$ = 364.01.

Example 5: 2-methoxyethyl 7-chloro-1-cyclopropyl-6-fluoro-4-oxo-1,4-dihydroquinoline-3-carboxylate (Compound 152)

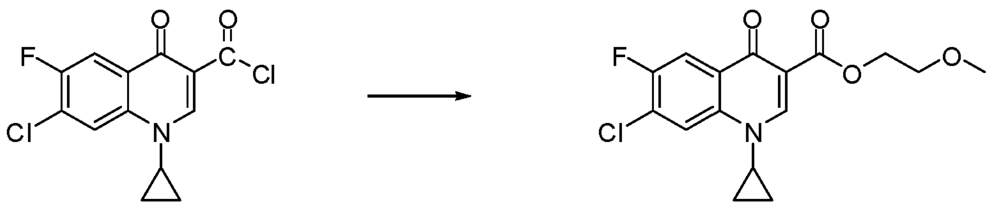

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 7-chloro-1-cyclopropyl-6-fluoro-4-oxo-1,4-dihydroquinoline-3-carbonyl chloride (2.85 g, 0.0095 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (2.83 g, 88% yield).

LC/MS$[M+H]^+$= 340.08, $[M+Na]^+$ = 362.06, $[M+K]^+$ = 378.03.

Example 6: 3-hydroxypropyl 7-chloro-1-cyclopropyl-6-fluoro-4-oxo-1,4-dihydroquinoline-3-carboxylate (Compound 164)

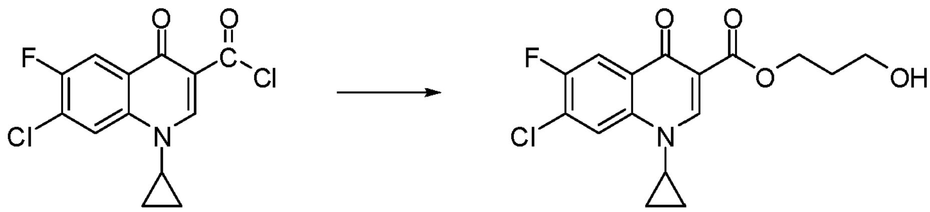

1,2-dihydroxypropane (0.76 g, 0.01 mol) and triethylamine (2.02 g, 0.02 mol) were dissolved sequentially in dichloromethane (20 mL) at room temperature. The reaction mixture was cooled to 0 °C in a cooling bath. Then the above mixture was added dropwise with a solution of 7-chloro-1-cyclopropyl-6-fluoro-4-oxo-1,4-dihydroquinoline-3-carbonyl chloride (2.85 g, 0.0095 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (2.22 g, 69% yield).

LC/MS$[M+H]^+$= 340.08, $[M+Na]^+$ = 362.06, $[M+K]^+$ = 378.03.

Example 7: 3-methoxypropyl 7-chloro-1-cyclopropyl-6-fluoro-4-oxo-1,4-dihydroquinoline-3-carboxylate (Compound 165)

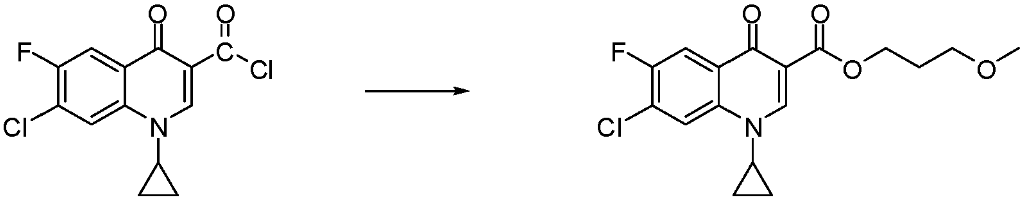

3-methoxy-1-propanol (0.90 g, 0.01 mol) and triethylamine (2.02 g, 0.02 mol) were dissolved sequentially in dichloromethane (20 mL) at room temperature. The reaction mixture was cooled to 0 °C in a cooling bath. Then the above mixture was added dropwise with a solution of 7-chloro-1-cyclopropyl-6-fluoro-4-oxo-1,4-dihydroquinoline-3-carbonyl chloride (2.85 g, 0.0095 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (15 mL × 3). The organic phases were combined, washed with water (15 mL × 1), washed with saturated brine (10 mL × 1), and dried over anhydrous magnesium sulfate. The crude product was precipitated under reduced pressure, and recrystallized from toluene to give the product (2.83 g, 84% yield).

LC/MS$[M+H]^+$= 354.09, $[M+Na]^+$ = 376.07, $[M+K]^+$ = 392.04.

Example 8: 2-methoxyethyl 1-cyclopropyl-6-fluoro-4-oxo-7-ethoxy-1,4-dihydroquinoline-3-carboxylate (Compound 282)

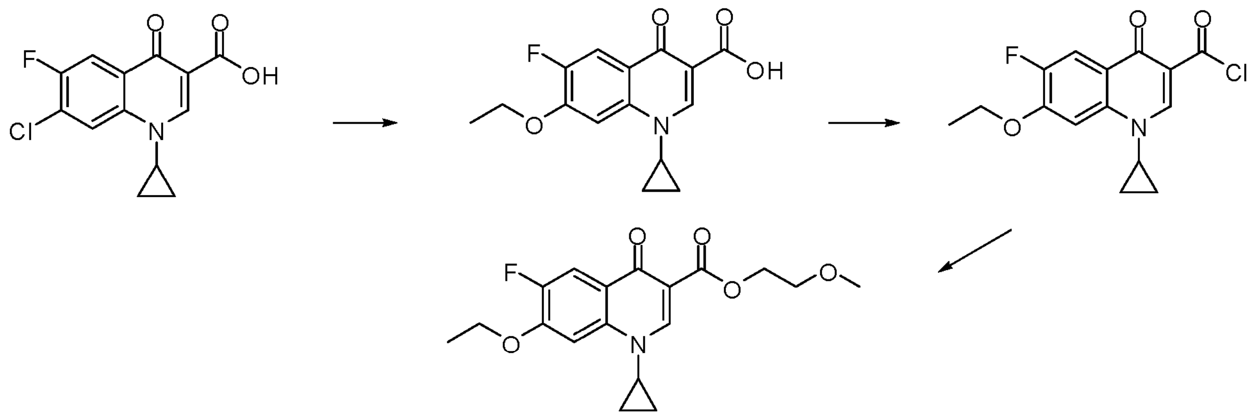

First reaction: 1-cyclopropyl-7-ethoxy-6-fluoro-4-oxo-1,4-dihydroquinoline-3-carboxylic acid

Sodium wire (0.46 g, 0.02 mol) was added portionwise to ethanol (2.30 g, 0.05 mol) at room temperature, and the mixture was heated to reflux for 3 h. The above solution was added sequentially with *N,N*-dimethylformamide (15 mL) and 7-chloro-1-cyclopropyl-6-fluoro-4-oxo-1,4-dihydroquinoline-3-carboxylic acid (2.82 g, 0.01 mol), and gradually heated to 90 °C to react for 5 h. After cooling to room temperature, the above reaction system was added with water (20 mL) and extracted with ethyl acetate (15 mL × 3). The organic phases were combined, washed with water (15 mL × 1), washed with saturated brine (10 mL × 1), and dried over anhydrous magnesium sulfate. The crude product was precipitated under reduced pressure, and purified by column chromatography (eluent: mixed solution of ethyl acetate, petroleum ether and formic acid (1:1:0.01)) to give the product (1.86 g, 64% yield).

LC/MS$[M+H]^+$= 292.1, $[M+Na]^+$ = 314.08, $[M+K]^+$ = 330.05.

Second reaction: 1-cyclopropyl-7-ethoxy-6-fluoro-4-oxo-1,4-dihydroquinoline-3-carbonyl chloride

1-cyclopropyl-7-ethoxy-6-fluoro-4-oxo-1,4-dihydroquinoline-3-carboxylic acid (1.80 g, 0.006 mol), dichloroethane (15 mL) and N,N-dimethylformamide (0.05 g) were added to a three-necked flask at room temperature, and the resulting mixture was added dropwise with thionyl chloride (2.5 g, 0.02 mol), and heated to reflux for 5 h. Then the heating was stopped, and the solvent and residual thionyl chloride were removed under reduced pressure to give the product (1.76 g, 95% yield).

Third reaction: 2-methoxyethyl 1-cyclopropyl-6-fluoro-4-oxo-7-ethoxy-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 1-cyclopropyl-7-ethoxy-6-fluoro-4-oxo-1,4-dihydroquinoline-3-carbonyl chloride (1.75 g, 0.0055 mol) in dichloromethane (10 mL). After the dropwise addition, the mixture was allowed to naturally warm to room temperature, and reacted for 4 h. The reaction mixture was added with a saturated aqueous sodium bicarbonate solution (8 mL) and extracted with dichloromethane (10 mL × 3). The organic phases were combined, washed with water (10 mL × 1), washed with saturated brine (10 mL × 1), and dried over anhydrous magnesium sulfate. The crude product was precipitated under reduced pressure, and recrystallized from toluene to give the product (1.65 g, 86% yield).
LC/MS$[M+H]^+$= 350.14, $[M+Na]^+$ = 372.12, $[M+K]^+$ = 388.09.

Example 9: 2-methoxyethyl 1-cyclopropyl-6-fluoro-4-oxo-7-(2,2,2-trifluoroethoxy)-1,4-dihydroquinoline-3-carboxylate (Compound 292)

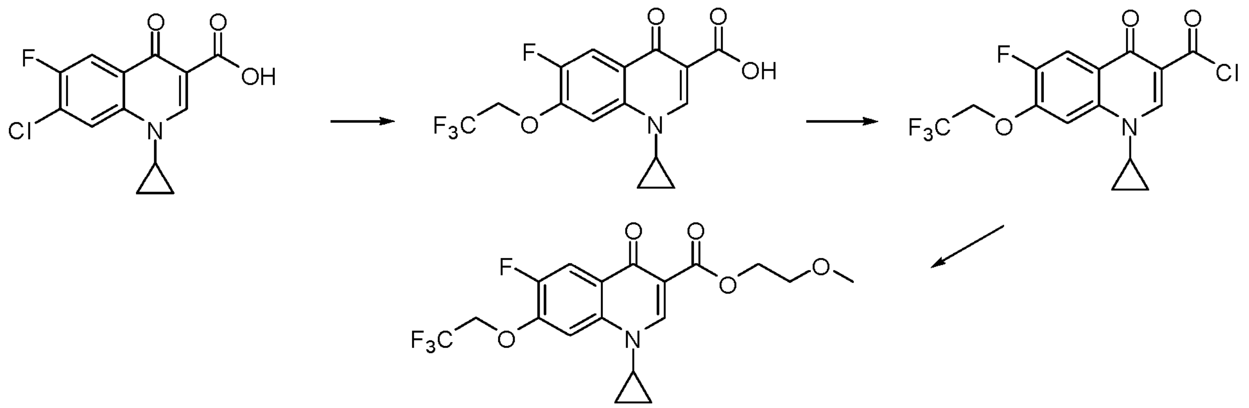

First reaction: 1-cyclopropyl-6-fluoro-4-oxo-7-(2,2,2-trifluoroethoxy)-1,4-dihydroquinoline-3-carboxylic acid

Sodium wire (0.46 g, 0.02 mol) was added portionwise to trifluoroethanol (4.00 g, 0.04 mol) at room temperature, and the mixture was heated to reflux for 5 h. The above solution was added sequentially with *N,N*-dimethylformamide (15 mL) and 7-chloro-1-cyclopropyl-6-fluoro-4-oxo-1,4-dihydroquinoline-3-carboxylic acid (2.82 g, 0.01 mol), and gradually heated to 90 °C to react for 5 h. After cooling to room temperature, the above reaction system was added with water (20 mL) and extracted with ethyl acetate (15 mL × 3). The organic phases were combined, washed with water (15 mL × 1), washed with saturated brine (10 mL × 1), and dried over anhydrous magnesium sulfate. The crude product was precipitated under reduced pressure, and purified by column chromatography (eluent: mixed solution of ethyl acetate, petroleum ether and formic acid (1:1:0.01)) to give the product (2.13 g, 62% yield).
LC/MS$[M+H]^+$= 346.07, $[M+Na]^+$ = 368.05, $[M+K]^+$ = 384.02.

Second reaction: 1-cyclopropyl-6-fluoro-4-oxo-7-(2,2,2-trifluoroethoxy)-1,4-dihydroquinoline-3-carbonyl chloride

1-cyclopropyl-6-fluoro-4-oxo-7-(2,2,2-trifluoroethoxy)-1,4-dihydroquinoline-3-carboxylic acid (2.10 g, 0.006 mol), dichloroethane (20 mL) and *N,N*-dimethylformamide (0.05 g) were added to a three-necked flask at room temperature, and the resulting mixture was added dopwise with thionyl chloride (2.5 g, 0.02 mol), and heated to reflux for 5 h. Then the heating was stopped, and the solvent and residual thionyl chloride were removed under reduced pressure to give the product (2.01 g, 92% yield).

Third reaction: 2-methoxyethyl 1-cyclopropyl-6-fluoro-4-oxo-7-(2,2,2-trifluoroethoxy)-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 1-cyclopropyl-6-fluoro-4-oxo-7-(2,2,2-trifluoroethoxy)-1,4-dihydroquinoline-3-carbonyl chloride (2.00 g, 0.0055 mol) in dichloromethane (10 mL). After the dropwise addition, the mixture was allowed to naturally warm to room temperature, and reacted for 4 h. The reaction mixture was added with a saturated aqueous sodium bicarbonate solution (8 mL) and extracted with dichloromethane (10 mL × 3). The organic phases were combined, washed with water (10 mL × 1), washed with saturated brine (10 mL × 1), and dried over anhydrous magnesium sulfate. The crude product was precipitated under reduced pressure, and recrystallized from toluene to give the product (1.82 g, 82% yield).
LC/MS$[M+H]^+$= 404.11, $[M+Na]^+$ = 426.09, $[M+K]^+$ = 442.06.

Example 10: 2-methoxyethyl 7-cyclopropoxy-1-cyclopropyl-6-fluoro-4-oxo-1,4-dihydroquinoline-3-carboxylate (Compound 294*, this compound is not according to the current invention)

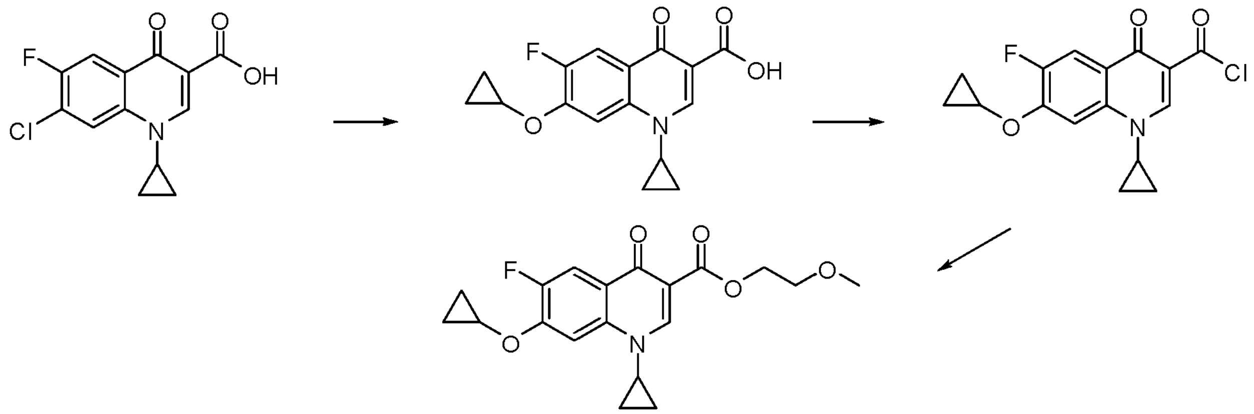

First reaction: 7-cyclopropyl-1-cyclopropyl-6-fluoro-4-oxo-1,4-dihydroquinoline-3-carboxylic acid

Sodium wire (0.46 g, 0.02 mol) was added portionwise to cyclopropanol (2.32 g, 0.04 mol) at room temperature, and the mixture was heated to reflux for 6 h. The above solution was added sequentially with *N,N*-dimethylformamide (15 mL) and 7-chloro-1-cyclopropyl-6-fluoro-4-oxo-1,4-dihydroquinoline-3-carboxylic acid (2.82 g, 0.01 mol), and gradually heated to 90 °C to react for 5 h. After cooling to room temperature, the above reaction system was added with water (20 mL) and extracted with ethyl acetate (15 mL × 3). The organic phases were combined, washed with water (15 mL × 1), washed with saturated brine (10 mL × 1), and dried over anhydrous magnesium sulfate. The crude product was precipitated under reduced pressure, and purified by column chromatography (eluent: mixed solution of ethyl acetate, petroleum ether and formic acid (1:1:0.01)) to give the product (1.57 g, 52% yield).
LC/MS$[M+H]^+$= 304.1, $[M+Na]^+$ = 326.08, $[M+K]^+$ = 342.05.

Second reaction: 7-cyclopropyl-1-cyclopropyl-6-fluoro-4-oxo-1,4-dihydroquinoline-3-carbonyl chloride

7-cyclopropyl-1-cyclopropyl-6-fluoro-4-oxo-1,4-dihydroquinoline-3-carboxylic acid (1.56 g, 0.005 mol), dichloroethane (20 mL) and N,N-dimethylformamide (0.05 g) were added to a three-necked flask at room temperature, and the resulting mixture was added dropwise with thionyl chloride (2.5 g, 0.02 mol), and heated to reflux for 5 h. Then the heating was stopped, and the solvent and residual thionyl chloride were removed under reduced pressure to give the product (1.51 g, 94% yield).

Third reaction: 2-methoxyethyl 7-cyclopropoxy-1-cyclopropyl-6-fluoro-4-oxo-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the

above mixture was added dropwise with a solution of 7-cyclopropyl-1-cyclopropyl-6-fluoro-4-oxo-1,4-dihydroquinoline-3-carbonyl chloride (1.50 g, 0.0046 mol) in dichloromethane (10 mL). After the dropwise addition, the mixture was allowed to naturally warm to room temperature, and reacted for 4 h. The reaction mixture was added with a saturated aqueous sodium bicarbonate solution (8 mL) and extracted with dichloromethane (10 mL × 3). The organic phases were combined, washed with water (10 mL × 1), washed with saturated brine (10 mL × 1), and dried over anhydrous magnesium sulfate. The crude product was precipitated under reduced pressure, and recrystallized from toluene to give the product (1.46 g, 88% yield). LC/MS$[M+H]^+$= 362.14, $[M+Na]^+$ = 384.12, $[M+K]^+$ = 400.09.

Example 11: 2-methoxyethyl 1-cyclopropyl-6-fluoro-7-methylthio-4-oxo-1,4-dihydroquinoline-3-carboxylate (Compound 381)

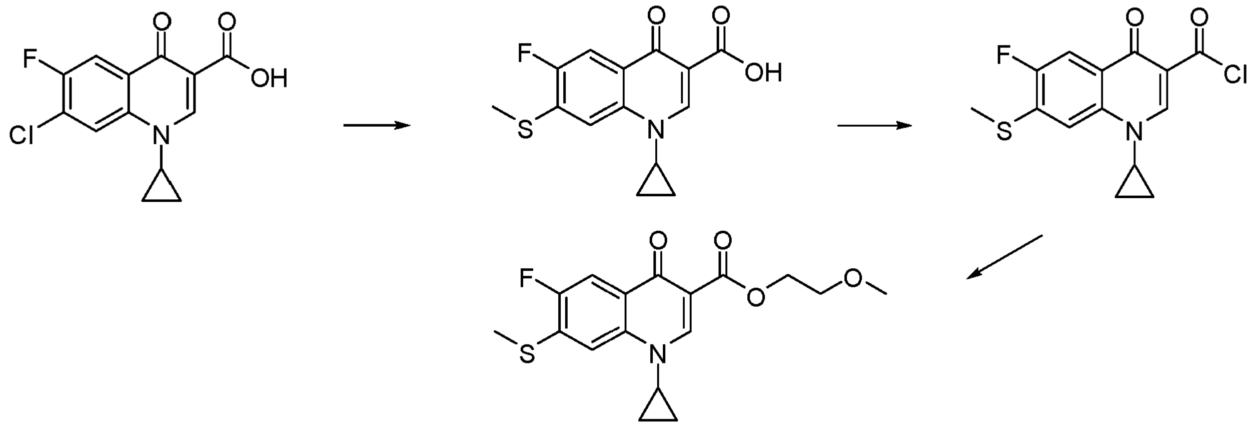

First reaction: 1-cyclopropyl-6-fluoro-7-methylthio-4-oxo-1,4-dihydroquinoline-3-carboxylic acid

Sodium thiomethoxide (1.40 g, 0.02 mol) and 7-chloro-1-cyclopropyl-6-fluoro-4-oxo-1,4-dihydroquinoline-3-carboxylic acid (2.82 g, 0.01 mol) were sequentially dissolved in N,N-dimethylformamide (15 mL) at room temperature, and the resulting mixture was gradually heated to 90 °C to react for 6 h. After cooling to room temperature, the above reaction system was added with water (20 mL) and extracted with ethyl acetate (15 mL × 3). The organic phases were combined, washed with water (15 mL × 1), washed with saturated brine (10 mL × 1), and dried over anhydrous magnesium sulfate. The crude product was precipitated under reduced pressure, and purified by column chromatography (eluent: mixed solution of ethyl acetate, petroleum ether and formic acid (1:1:0.01)) to give the product (1.64 g, 56% yield).

LC/MS$[M+H]^+$= 294.06, $[M+Na]^+$ = 316.04, $[M+K]^+$ = 332.01.

Second reaction: 1-cyclopropyl-6-fluoro-7-methylthio-4-oxo-1,4-dihydroquinoline-3-carbonyl chloride

1-cyclopropyl-6-fluoro-7-methylthio-4-oxo-1,4-dihydroquinoline-3-carboxylic acid (1.62 g, 0.0055 mol), dichloroethane (20 mL) and N,N-dimethylformamide (0.05 g) were added to a three-necked flask at room temperature, and the resulting mixture was added dropwise with thionyl chloride (2.5 g, 0.02 mol), and heated to reflux for 5 h. Then the heating was stopped, and the solvent and residual thionyl chloride were removed under reduced pressure to give the product (1.64 g, 96% yield).

Third reaction: 2-methoxyethyl 1-cyclopropyl-6-fluoro-7-methylthio-4-oxo-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 1-cyclopropyl-6-fluoro-7-methylthio-4-oxo-1,4-dihydroquinoline-3-carbonyl chloride (1.60 g, 0.0051 mol) in dichloromethane (10 mL). After the dropwise addition, the mixture was allowed to naturally warm to room temperature, and reacted for 4 h. The reaction mixture was added with a saturated aqueous sodium bicarbonate solution (8 mL) and extracted with dichloromethane (10 mL × 3). The organic phases were combined, washed with water (10 mL × 1), washed with saturated brine (10 mL × 1), and dried over anhydrous magnesium sulfate. The crude product was precipitated under reduced pressure, and recrystallized from toluene to give the product (1.52 g, 85% yield).

LC/MS$[M+H]^+$= 352.1, $[M+Na]^+$ = 374.08, $[M+K]^+$ = 390.05.

Example 12: 2-methoxyethyl 1-cyclopropyl-7-(diethylamino)-6-fluoro-4-oxo-1,4-dihydroquinoline-3-carboxylate (Compound 420)

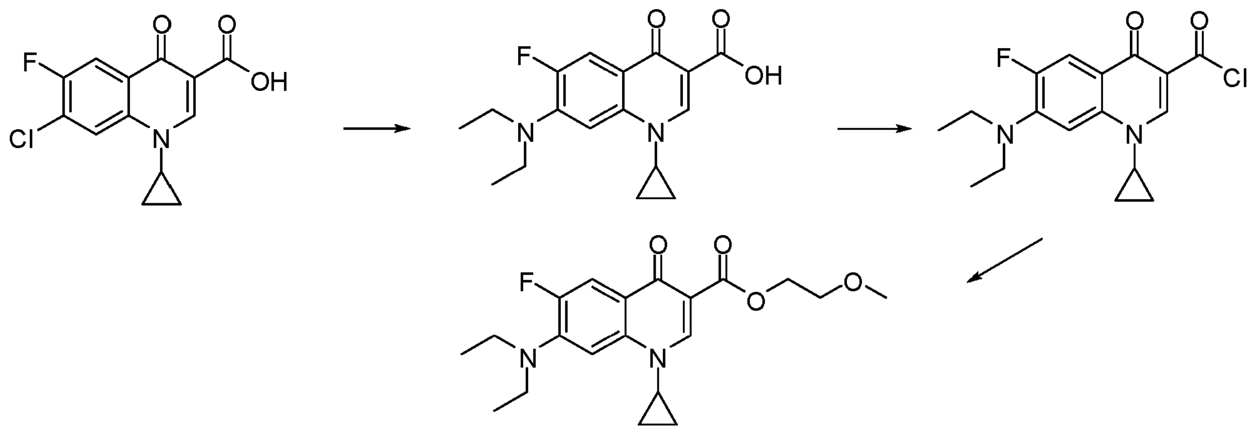

First reaction: 1-cyclopropyl-7-(diethylamino)-6-fluoro-4-oxo-1,4-dihydroquinoline-3-carboxylic acid

Diethylamine (2.19 g, 0.03 mol) and 7-chloro-1-cyclopropyl-6-fluoro-4-oxo-1,4-dihydroquinoline-3-carboxylic acid (2.82 g, 0.01 mol) were sequentially dissolved in dimethyl sulfoxide (15 mL) at room temperature, and the resulting mixture was gradually heated to 90 °C to react for 6 h. After cooling to room temperature, the above reaction system was added with water (20 mL) and extracted with ethyl acetate (10 mL × 3). The organic phases were combined, washed with water (15 mL × 2), washed with saturated brine (10 mL × 1), and dried over anhydrous magnesium sulfate. The crude product was precipitated under reduced pressure, and purified by column chromatography (eluent: mixed solution of ethyl acetate, petroleum ether and formic acid (1:1:0.01)) to give the product (2.59 g, 81% yield).

LC/MS$[M+H]^+$= 319.15, $[M+Na]^+$ = 341.13, $[M+K]^+$ = 357.1.

Second reaction: 1-cyclopropyl-7-(diethylamino)-6-fluoro-4-oxo-1,4-dihydroquinoline-3-carbonyl chloride

1-cyclopropyl-7-(diethylamino)-6-fluoro-4-oxo-1,4-dihydroquinoline-3-carboxylic acid (2.5 g, 0.008 mol), dichloroethane (15 mL) and N,N-dimethylformamide (0.05 g) were added to a three-necked flask at room temperature, and the resulting mixture was added dropwise with thionyl chloride (2.5 g, 0.02 mol), and heated to reflux for 4 h. Then the heating was stopped, and the solvent and residual thionyl chloride were removed under reduced pressure to give the product (2.39 g, 89% yield).

Third reaction: 2-methoxyethyl 1-cyclopropyl-7-(diethylamino)-6-fluoro-4-oxo-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. Then the above mixture was added dropwise with a solution of 1-cyclopropyl-7-(diethylamino)-6-fluoro-4-oxo-1,4-dihydroquinoline-3-carbonyl chloride (2.06 g, 0.006 mol) in dichloromethane (10 mL). After the dropwise addition, the mixture was allowed to naturally warm to room temperature, and reacted for 4 h. The reaction mixture was added with a saturated aqueous sodium bicarbonate solution (10 mL) and extracted with dichloromethane (10 mL × 3). The organic phases were combined, washed with water (10 mL × 1), washed with saturated brine (10 mL × 1), and dried over anhydrous magnesium sulfate. The crude product was precipitated under reduced pressure, and recrystallized from toluene to give the product (1.95 g, 86% yield). LC/MS$[M+H]^+$= 377.19, $[M+Na]^+$ = 399.17, $[M+K]^+$ = 415.14

Example 13: 2-methoxyethyl 1-cyclopropyl-6-fluoro-7-morpholinyl-4-oxo-1,4-dihydroquinoline-3-carboxylate (Compound 426*, not according to the current invention)

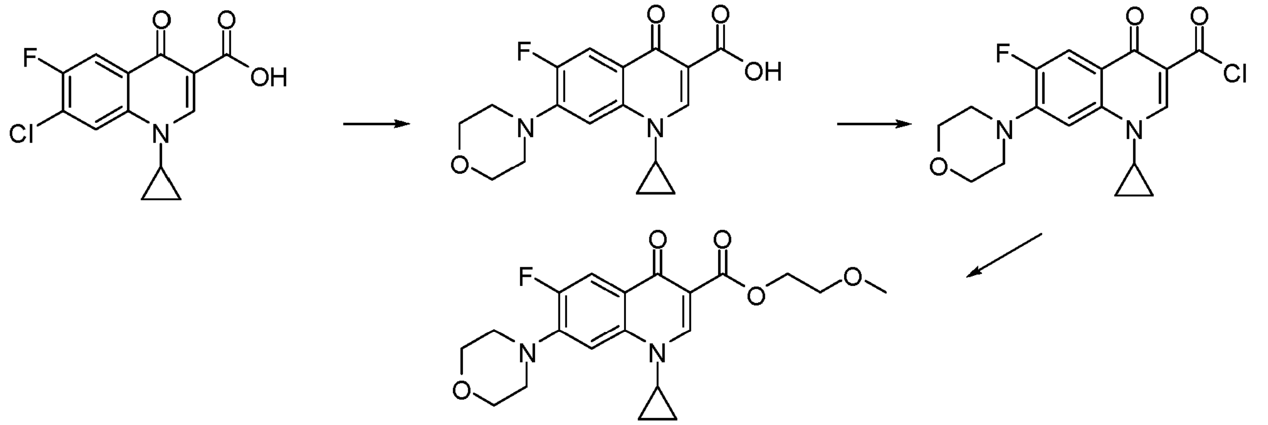

First reaction: 1-cyclopropyl-6-fluoro-7-morpholinyl-4-oxo-1,4-dihydroquinoline-3-carboxylic acid

Morpholine (1.76 g, 0.02 mol), 7-chloro-1-cyclopropyl-6-fluoro-4-oxo-1,4-dihydroquinoline-3-carboxylic acid (2.82 g, 0.01 mol) and triethylamine (2.02 g, 0.02 mol) were sequentially dissolved in dimethyl sulfoxide (15 mL) at room temperature, and the resulting mixture was gradually heated to 120 °C to react for 6 h. After cooling to room temperature, the above reaction system was added with water (20 mL) and extracted with ethyl acetate (15 mL × 3). The organic phases were combined, washed with water (15 mL × 2), washed with saturated brine (10 mL × 1), and dried over anhydrous magnesium sulfate. The crude product was precipitated under reduced pressure, and purified by column chromatography (eluent: mixed solution of ethyl acetate, petroleum ether and formic acid (1:1:0.01)) to give the product (2.62 g, 79% yield).
LC/MS$[M+H]^+$ = 333.13, $[M+Na]^+$ = 355.11, $[M+K]^+$ = 371.08.

Second reaction: 1-cyclopropyl-6-fluoro-7-morpholinyl-4-oxo-1,4-dihydroquinoline-3-carbonyl chloride

1-cyclopropyl-6-fluoro-7-morpholinyl-4-oxo-1,4-dihydroquinoline-3-carboxylic acid (2.66 g, 0.008 mol), dichloroethane (20 mL) and N,N-dimethylformamide (0.05 g) were added to a three-necked flask at room temperature, and the resulting mixture was added dropwise with thionyl chloride (2.5 g, 0.02 mol), and heated to reflux for 5 h. Then the heating was stopped, and the solvent and residual thionyl chloride were removed under reduced pressure to give the product (2.6 g, 92% yield).

Third reaction: 2-methoxyethyl 1-cyclopropyl-6-fluoro-7-morpholinyl-4-oxo-1,4-dihydroquinoline-3-carboxylate

2-methoxyethanol (0.76 g, 0.01 mol) and triethylamine (2.02 g, 0.02 mol) were sequentially dissolved in dichloromethane (20 mL) at room temperature. The reaction mixture was cooled to 0 °C in a cooling bath. The the above mixture was added dropwise with a solution of 1-cyclopropyl-6-fluoro-7-morpholinyl-4-oxo-1,4-dihydroquinoline-3-carbonyl chloride (2.10 g, 0.006 mol) in dichloromethane (10 mL). After the dropwise addition, the mixture was warmed to 35 °C, and reacted for 4 h. The reaction mixture was added with a saturated aqueous sodium bicarbonate solution (8 mL) and extracted with dichloromethane (10 mL × 3). The organic phases were combined, washed with water (10 mL × 1), washed with saturated brine (10 mL × 1), and dried over anhydrous magnesium sulfate. The crude product was precipitated under reduced pressure, and recrystallized from toluene to give the product (1.9 g, 81% yield).
LC/MS$[M+H]^+$= 391.17, $[M+Na]^+$ = 413.15, $[M+K]^+$ = 429.12.
Other compounds of the present disclosure were synthesized with reference to the methods described above.
The structural characterization data of other compounds of the formula (I) are as follows (in the following table, the compounds labelled with a "*" are not according to the current invention):

| Compound No. | Compound structure | Structural characterization data |
|---|---|---|
| 2 | 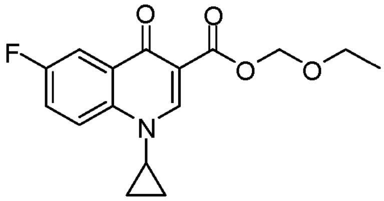 | LC/MS[M+H]$^{+}$= 306.12, [M+Na]$^{+}$ = 328.1, [M+K]$^{+}$ = 344.07 |
| 18 | 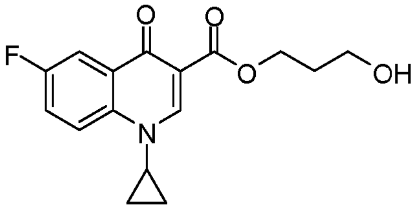 | LC/MS[M+H]$^{+}$= 306.12, [M+Na]$^{+}$ = 328.1, [M+K]$^{+}$ = 344.07 |
| 44 | 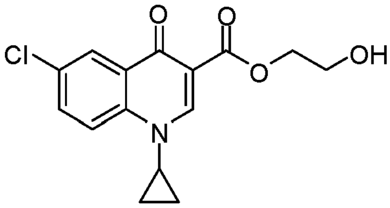 | LC/MS[M+H]$^{+}$= 308.07, [M+Na]$^{+}$ = 330.05, [M+K]$^{+}$ = 346.02 |
| 143 | 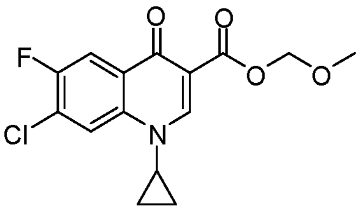 | LC/MS[M+H]$^{+}$= 326.06, [M+Na]$^{+}$ = 348.04, [M+K]$^{+}$ = 364.01 |
| 144 | 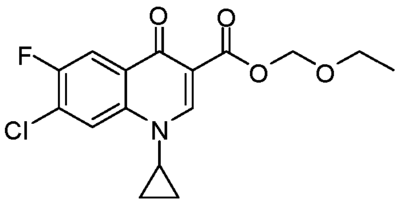 | LC/MS[M+H]$^{+}$= 340.08, [M+Na]$^{+}$ = 362.06, [M+K]$^{+}$ = 378.03 |
| 153 | 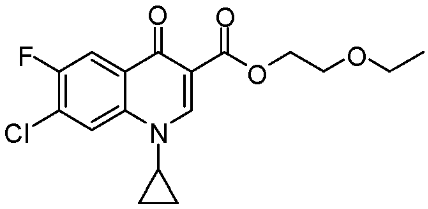 | LC/MS[M+H]$^{+}$= 354.09, [M+Na]$^{+}$ = 376.07, [M+K]$^{+}$ = 392.04 |
| 160 | 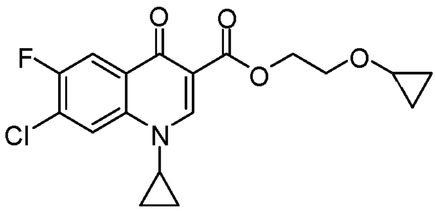 | LC/MS[M+H]$^{+}$= 366.09, [M+Na]$^{+}$ = 388.07, [M+K]$^{+}$ = 404.04 |

(continued)

| Compound No. | Compound structure | Structural characterization data |
|---|---|---|
| **177** | 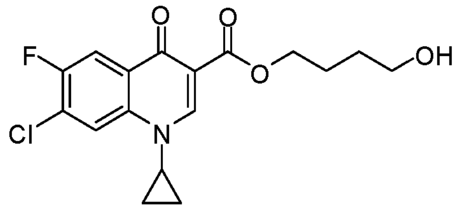 | **LC/MS[M+H]$^+$= 354.09, [M+Na]$^+$ = 376.07, [M+K]$^+$ = 392.04** |
| **178** | 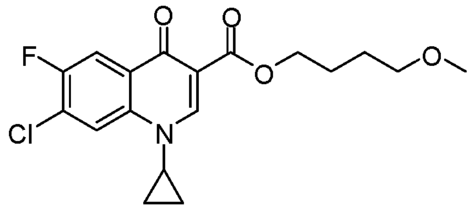 | **LC/MS[M+H]$^+$= 368.11, [M+Na]$^+$ = 390.09, [M+K]$^+$ = 406.06** |
| **194** | 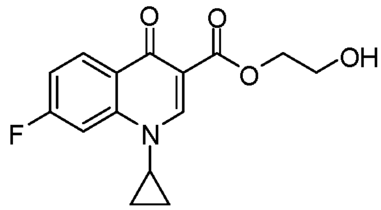 | **LC/MS[M+H]$^+$= 292.1, [M+Na]$^+$ = 314.08, [M+K]$^+$ = 330.05** |
| **195** | 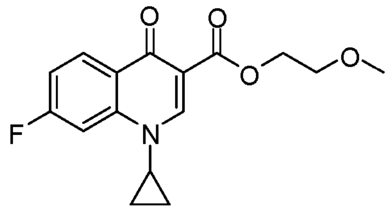 | **LC/MS[M+H]$^+$= 306.12, [M+Na]$^+$ = 328.1, [M+K]$^+$ = 344.07** |
| **207** | 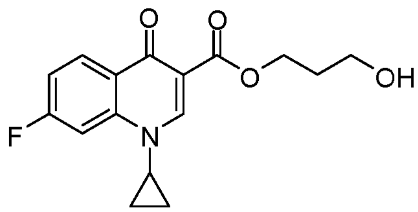 | **LC/MS[M+H]$^+$= 306.12, [M+Na]$^+$ = 328.1, [M+K]$^+$ = 344.07** |
| **208** | 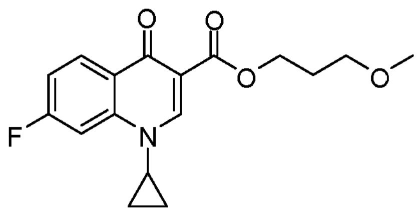 | **LC/MS[M+H]$^+$= 320.13, [M+Na]$^+$ = 342.11, [M+K]$^+$ = 358.08** |
| **264** | 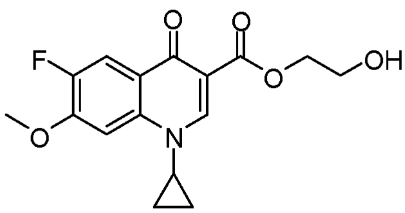 | **LC/MS[M+H]$^+$= 322.11, [M+Na]$^+$ = 344.09, [M+K]$^+$ = 360.06** |

(continued)

| Compound No. | Compound structure | Structural characterization data |
|---|---|---|
| **265** | 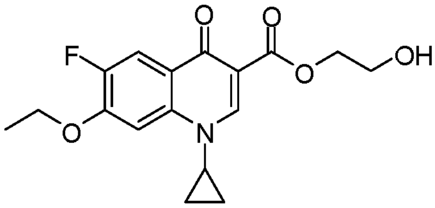 | **LC/MS[M+H]$^{+}$= 336.13, [M+Na]$^{+}$ = 358.11, [M+K]$^{+}$ = 374.08** |
| **270** | 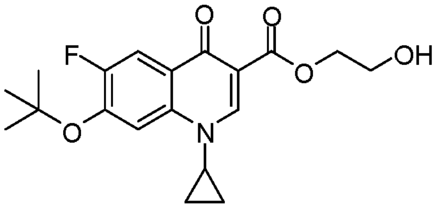 | **LC/MS[M+H]$^{+}$= 364.16, [M+Na]$^{+}$ = 386.14, [M+K]$^{+}$ = 402.11** |
| **275** | 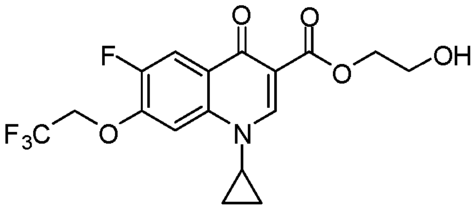 | **LC/MS[M+H]$^{+}$= 390.1, [M+Na]$^{+}$ = 412.08, [M+K]$^{+}$ = 428.05** |
| **281** | 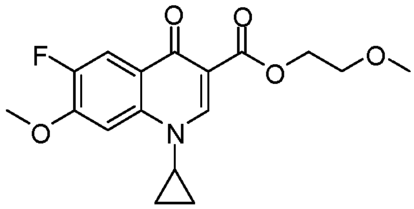 | **LC/MS[M+H]$^{+}$= 336.13, [M+Na]$^{+}$ = 358.11, [M+K]$^{+}$ = 374.08** |
| **287** | 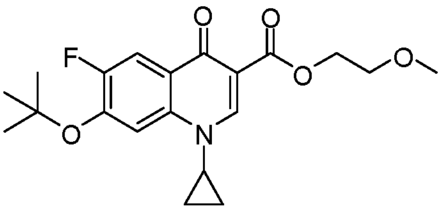 | **LC/MS[M+H]$^{+}$= 378.17, [M+Na]$^{+}$ = 400.15, [M+K]$^{+}$ = 416.12** |
| **296*** | 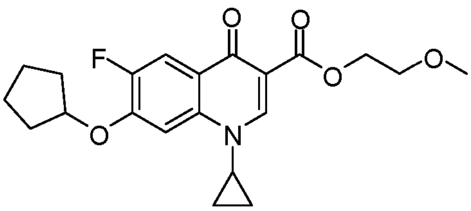 | **LC/MS[M+H]$^{+}$ = 390.17, [M+Na]$^{+}$ = 412.15, [M+K]$^{+}$ = 428.12** |
| **300** | 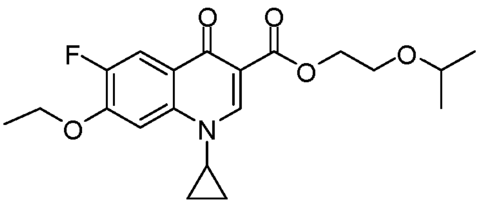 | **LC/MS[M+H]$^{+}$ = 378.17, [M+Na]$^{+}$ = 400.15, [M+K]$^{+}$ = 416.12** |

(continued)

| Compound No. | Compound structure | Structural characterization data |
|---|---|---|
| 307 | 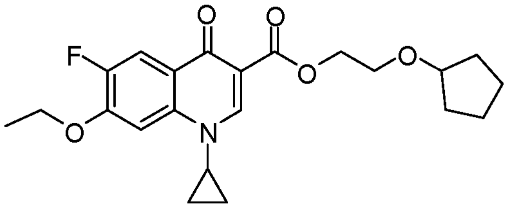 | LC/MS$[M+H]^+$ = 404.19, $[M+Na]^+$ = 426.17, $[M+K]^+$ = 442.14 |
| 309 | 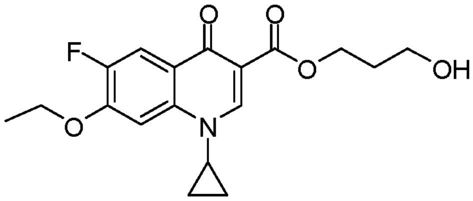 | LC/MS$[M+H]^+$ = 350.14, $[M+Na]^+$ = 372.12, $[M+K]^+$ = 388.09 |
| 312 | 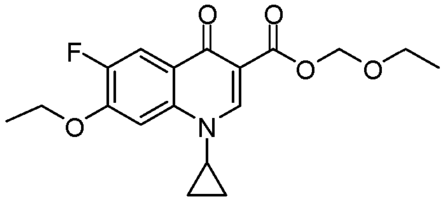 | LC/MS$[M+H]^+$ = 350.14, $[M+Na]^+$ = 372.12, $[M+K]^+$ = 388.09 |
| 315 | 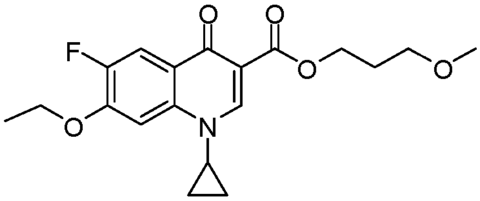 | LC/MS$[M+H]^+$ = 364.16, $[M+Na]^+$ = 386.14, $[M+K]^+$ = 402.11 |
| 320 | 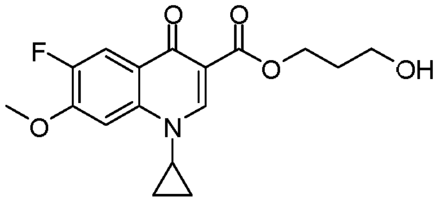 | LC/MS$[M+H]^+$ = 336.13, $[M+Na]^+$ = 358.11, $[M+K]^+$ = 374.08 |
| 323 | 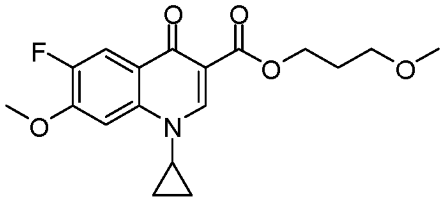 | LC/MS$[M+H]^+$ = 350.14, $[M+Na]^+$ = 372.12, $[M+K]^+$ = 388.09 |
| 326 | 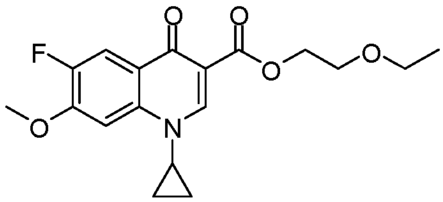 | LC/MS$[M+H]^+$ = 350.14, $[M+Na]^+$ = 372.12, $[M+K]^+$ = 388.09 |

(continued)

| Compound No. | Compound structure | Structural characterization data |
|---|---|---|
| 329 | 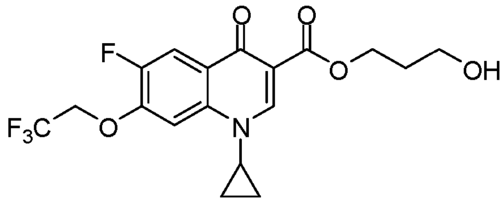 | LC/MS[M+H]$^+$ = 404.11, [M+Na]$^+$ = 426.09, [M+K]$^+$ = 442.06 |
| 335 | 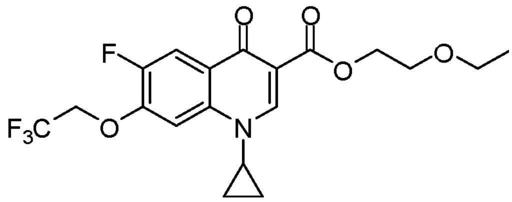 | LC/MS[M+H]$^+$ = 418.13, [M+Na]$^+$ = 440.11, [M+K]$^+$ = 456.08 |
| 342 | 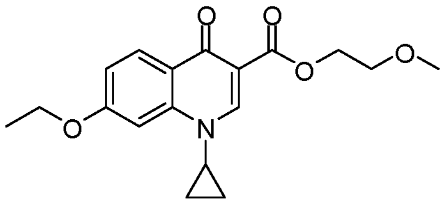 | LC/MS[M+H]$^+$ = 332.15, [M+Na]$^+$ = 354.13, [M+K]$^+$ = 370.1 |
| 353 | 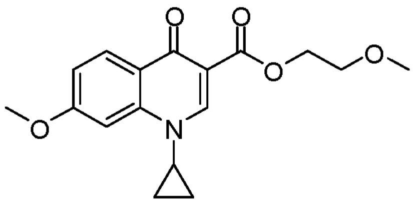 | LC/MS[M+H]$^+$ = 318.14, [M+Na]$^+$ = 340.12, [M+K]$^+$ = 356.09 |
| 360 | 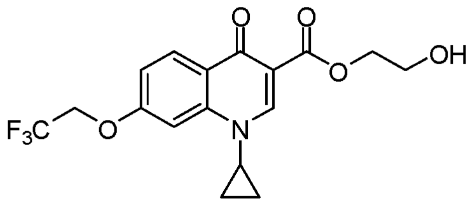 | LC/MS[M+H]$^+$ = 372.11, [M+Na]$^+$ = 394.09, [M+K]$^+$ = 410.06 |
| 364 | 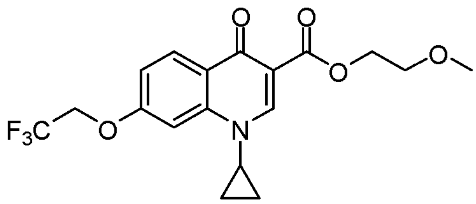 | LC/MS[M+H]$^+$ = 386.12, [M+Na]$^+$ = 408.1, [M+K]$^+$ = 424.07 |
| 371 | 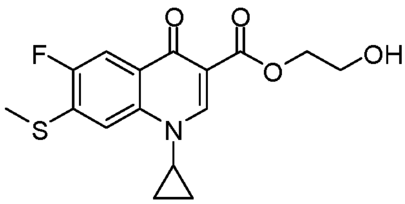 | LC/MS[M+H]$^+$ = 338.09, [M+Na]$^+$ = 360.07, [M+K]$^+$ = 376.04 |

(continued)

| Compound No. | Compound structure | Structural characterization data |
|---|---|---|
| **372** | 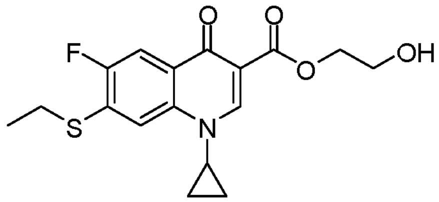 | **LC/MS[M+H]$^+$ = 352.1, [M+Na]$^+$ = 374.08, [M+K]$^+$ = 390.05** |
| **382** | 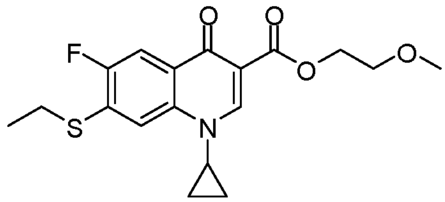 | **LC/MS[M+H]$^+$ = 366.12, [M+Na]$^+$ = 388.1, [M+K]$^+$ = 404.07** |
| **390** | 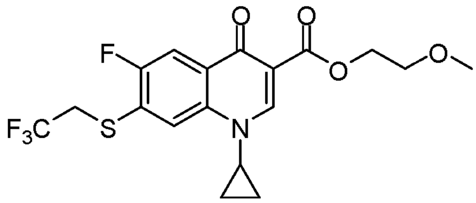 | **LC/MS[M+H]$^+$ = 420.09, [M+Na]$^+$ = 442.07, [M+K]$^+$ = 458.04** |
| **397** | 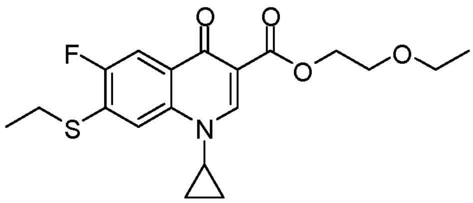 | **LC/MS[M+H]$^+$ = 380.14, [M+Na]$^+$ = 402.12, [M+K]$^+$ = 418.09** |
| **400** | 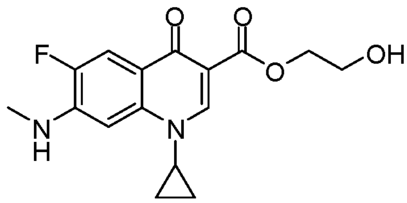 | **LC/MS[M+H]$^+$ = 321.13, [M+Na]$^+$ = 343.11, [M+K]$^+$ = 359.08** |
| **401** | 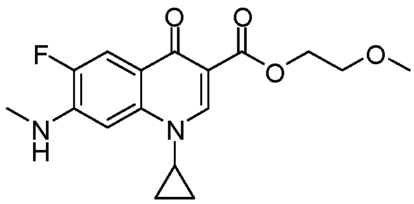 | **LC/MS[M+H]$^+$ = 335.14, [M+Na]$^+$ = 357.12, [M+K]$^+$ = 373.09** |
| **407** | 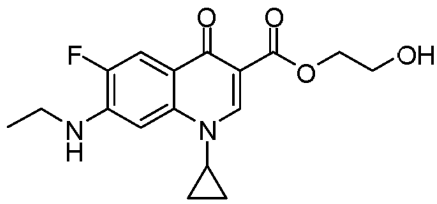 | **LC/MS[M+H]$^+$ = 335.14, [M+Na]$^+$ = 357.12, [M+K]$^+$ = 373.09** |

(continued)

| Compound No. | Compound structure | Structural characterization data |
|---|---|---|
| **408** | 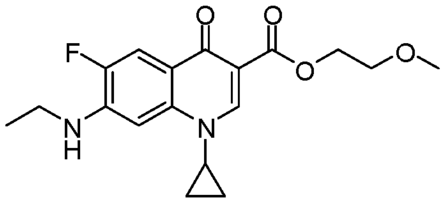 | **LC/MS[M+H]$^+$ = 349.16, [M+Na]$^+$ = 371.14, [M+K]$^+$ = 387.11** |
| **413** | 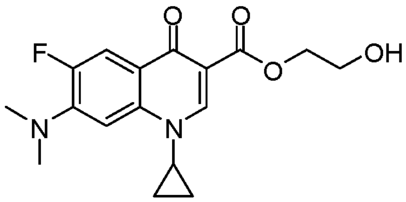 | **LC/MS[M+H]$^+$ = 335.14, [M+Na]$^+$ = 357.12, [M+K]$^+$ = 373.09** |
| **414** | 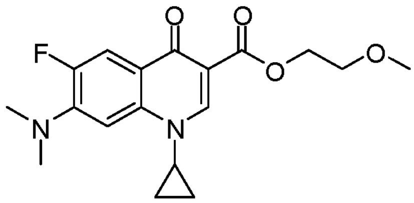 | **LC/MS[M+H]$^+$ = 349.16, [M+Na]$^+$ = 371.14, [M+K]$^+$ = 387.11** |
| **419** | 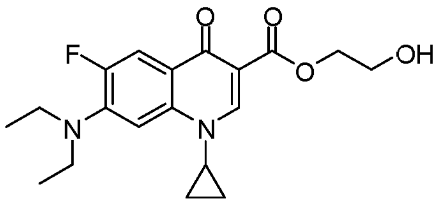 | **LC/MS[M+H]$^+$ = 363.17, [M+Na]$^+$ = 385.15, [M+K]$^+$ = 401.12** |
| **425*** | 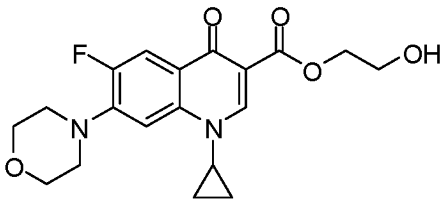 | **LC/MS[M+H]$^+$ = 377.15, [M+Na]$^+$ = 399.13, [M+K]$^+$ = 415.1** |
| **431*** | 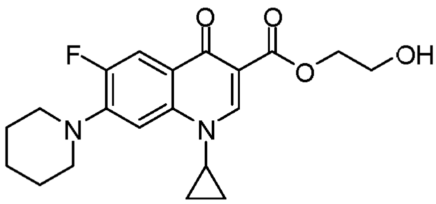 | **LC/MS[M+H]$^+$ = 375.17, [M+Na]$^+$ = 397.15, [M+K]$^+$ = 413.12** |
| **432*** | 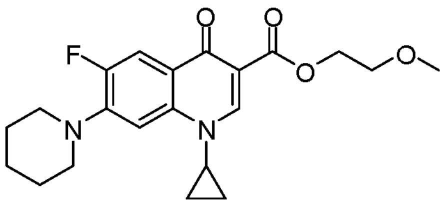 | **LC/MS[M+H]$^+$ = 389.19, [M+Na]$^+$ = 411.17, [M+K]$^+$ = 427.14** |
| The compounds of No. 296, 425, 431 and 432 in the above table are not part of the present invention as indicated by the *. | | |

Formulation examples

In the following examples, all percentages are by weight and all dosage forms are prepared by conventional methods. In the following, compounds indicated by a "*" are not according to the current invention.

Example 14:

In this example, the compound obtained in the above example was used to prepare a wettable powder, which was specifically prepared from the starting materials of the following ratios:
Compound 151, 60.0%; dodecylphenol polyethoxy glycol ether, 4.0%; sodium lignosulfonate, 5.0%; sodium aluminosilicate, 6.0%; and montmorillonite (calcined), 25.0%.

Example 15:

In this example, the compound obtained in the above example was used to prepare a granule, which was specifically prepared from the starting materials of the following ratios:
Compound 152, 10.0%; and the other components are sodium dodecyl sulfate, 2%; calcium lignosulfonate, 6%; potassium chloride, 10%; polydimethylsiloxane, 1%; and soluble starch making up the rest.

Example 16:

In this example, the compound obtained in the above examples was used to prepare an extruded pill, which was specifically prepared from the starting materials of the following ratios:
Compound 164, 25.0%; anhydrous calcium sulfate, 10.0%; crude calcium lignosulfonate, 5.0%; sodium alkyl naphthalene sulfonate, 1.0%; and calcium/magnesium bentonite, 59.0%.

Example 17:

In this example, the compound obtained in the above examples was used to prepare an emulsifiable concentrate, which was specifically prepared from the starting materials of the following ratios:
Compound 165, 25.0%; solvent 150, 60%; PEG 400, 5%; Rhodacal 70/B, 3%; and Rhodameen RAM/7, 7%.

Example 18:

In this example, the compound obtained in the above example was used to prepare an aqueous suspension, which was specifically prepared from the starting materials of the following ratios:
Compound 282, 30%; POE polystyrene phenyl ether sulfate, 5.0%; xanthan gum, 0.5%; polyethylene glycol, 5%; triethanolamine, 1%; sorbitol, 0.5%; and water making up the rest.

Assay for biological activity

The compound of the present disclosure shows good activity against various bacterial germs in the agricultural field.

Example 19: assay for bactericide activity

The compounds of the present disclosure were tested for *in vitro* bacteriostatic activity or *in vivo* protective effect against various bacterial diseases in plants, and for effect of improving the growth and development of crops. The results of the assay for bactericidal activity and the effect of improving the growth and development of crops are shown in the following examples.

1. Assay for *in vitro* bactericidal activity

The test method is as follows: the agent was formulated and diluted to a range of concentrations with a suitable solvent (such as acetone, methanol, N,N-dimethylformamide and dimethylsulfoxide, and was selected according to its capability of dissolving the sample). Under aseptic conditions, equal amounts of NB culture medium were added to test tubes, and fixed amounts of the sample solutions were pipetted into the above test tubes in an ascending order of concentrations, respectively. The mixtures were well mixed and added with equal amounts of a bacterial suspension in log

phase. Each step of the procedure was repeated 4 times. After mixing, the mixture samples were incubated in the dark in a shaking incubator at 25 °C. The OD values were measured when the bacteria were in log phase.

(1) The test results of the *in vitro* bacteriostatic activity (shown as inhibitory rates) of part of the compounds against the pathogenic bacteria causing bacterial angular leaf spot in cucumbers are as follows:

At a dosage of 5 ppm, the compounds having an inhibitory rate of no less than 90% for the pathogenic bacteria causing bacterial angular leaf spot in cucumbers comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 2% and 48% for the pathogenic bacteria causing bacterial angular leaf spot in cucumbers, respectively.

At a dosage of 1 ppm, the compounds having an inhibitory rate of no less than 90% for the pathogenic bacteria causing bacterial angular leaf spot in cucumbers comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 18, 44, 143, 144, 160, 194, 353, 360 and 364. At this dosage, the control agents oxine-copper and zhongshengmycin have inhibitory rates of 0% and 21% for the pathogenic bacteria causing bacterial angular leaf spot in cucumbers, respectively.

(2) The test results of the *in vitro* bacteriostatic activity (shown as inhibitory rates) of part of the compounds against the pathogenic bacteria causing bacterial wilt in tobacco are as follows:

At a dosage of 5 ppm, the compounds having an inhibitory rate of no less than 90% for the pathogenic bacteria causing bacterial wilt in tobacco comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 5% and 51% for the pathogenic bacteria causing bacterial wilt in tobacco, respectively.

At a dosage of 1 ppm, the compounds having an inhibitory rate of no less than 90% for the pathogenic bacteria causing bacterial wilt in tobacco comprise: 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 10, 18, 44, 143, 144, 160, 194, 353, 360 and 364. At this dosage, the control agents oxine-copper and zhongshengmycin have inhibitory rates of 0% and 28% for the pathogenic bacteria causing bacterial wilt in tobacco, respectively.

(3) The test results of the *in vitro* bacteriostatic activity (shown as inhibitory rates) of part of the compounds against the pathogenic bacteria causing black shank in potatoes are as follows:

At a dosage of 5 ppm, the compounds having an inhibitory rate of no less than 90% for the pathogenic bacteria causing black shank in potatoes comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 3% and 55% for the pathogenic bacteria causing black shank in potatoes, respectively.

At a dosage of 1 ppm, the compounds having an inhibitory rate of no less than 90% for the pathogenic bacteria causing black shank in potatoes comprise: 45, 151, 152, 153, 164, 165, 177, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 10, 18, 44, 143, 144, 160, 178, 194, 353, 360 and 364. At this dosage, the control agents oxine-copper and zhongshengmycin have inhibitory rates of 0% and 38% for the pathogenic bacteria causing black shank in potatoes, respectively.

(4) The test results of the *in vitro* bacteriostatic activity (shown as inhibitory rates) of part of the compounds against the

pathogenic bacteria causing stem rot in sweet potatoes are as follows:

At a dosage of 5 ppm, the compounds having an inhibitory rate of no less than 90% for the pathogenic bacteria causing stem rot in sweet potatoes comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 7% and 46% for the pathogenic bacteria causing stem rot in sweet potatoes, respectively.

At a dosage of 1 ppm, the compounds having an inhibitory rate of no less than 90% for the pathogenic bacteria causing stem rot in sweet potatoes comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 18, 44, 143, 144, 160, 194, 353, 360 and 364. At this dosage, the control agents oxine-copper and zhongshengmycin have inhibitory rates of 2% and 25% for the pathogenic bacteria causing stem rot in sweet potatoes, respectively.

(5) The test results of the *in vitro* bacteriostatic activity (shown as inhibitory rates) of part of the compounds against the pathogenic bacteria causing bacterial blight in rice are as follows:

At a dosage of 5 ppm, the compounds having an inhibitory rate of no less than 90% for the pathogenic bacteria causing bacterial blight in rice comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 16% and 48% for the pathogenic bacteria causing bacterial blight in rice, respectively.

At a dosage of 1 ppm, the compounds having an inhibitory rate of no less than 90% for the pathogenic bacteria causing bacterial blight in rice comprise: 45, 151, 152, 153, 164, 165, 178, 195, 207, 208, 264, 265, 270, 275, 281, 282, 287, 292, 294*, 296*, 300, 307, 309, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 10, 18, 44, 143, 144, 160, 177, 194, 312, 353, 360 and 364. At this dosage, the control agents oxine-copper and zhongshengmycin have inhibitory rates of 9% and 30% for the pathogenic bacteria causing bacterial blight in rice, respectively.

(6) The test results of the *in vitro* bacteriostatic activity (shown as inhibitory rates) of part of the compounds against the pathogenic bacteria causing fruit blotch in watermelons are as follows:

At a dosage of 5 ppm, the compounds having an inhibitory rate of no less than 90% for the pathogenic bacteria causing fruit blotch in watermelons comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 3% and 52% for the pathogenic bacteria causing fruit blotch in watermelons, respectively.

At a dosage of 1 ppm, the compounds having an inhibitory rate of no less than 90% for the pathogenic bacteria causing fruit blotch in watermelons comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 18, 44, 143, 144, 160, 194, 353, 360 and 364. At this dosage, the control agents oxine-copper and zhongshengmycin have inhibitory rates of 0% and 29% for the pathogenic bacteria causing fruit blotch in watermelons, respectively.

(7) The test results of the *in vitro* bacteriostatic activity (shown as inhibitory rates) of part of the compounds against the pathogenic bacteria causing black rot in Chinese cabbages are as follows:

At a dosage of 5 ppm, the compounds having an inhibitory rate of no less than 90% for the pathogenic bacteria causing black rot in Chinese cabbages comprise: 1, 2, 10, 18, 44, 45, 143, 144, 151, 152, 153, 160, 164, 165, 177,

178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 5% and 46% for the pathogenic bacteria causing black rot in Chinese cabbages, respectively.

At a dosage of 1 ppm, the compounds having an inhibitory rate of no less than 90% for the pathogenic bacteria causing black rot in Chinese cabbages comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 400, 401, 407, 408, 413, 414, 419, 420, 425*, 426*, 431* and 432*; the compounds having an inhibitory rate of no less than 80% comprise: 1, 18, 44, 143, 144, 160, 194, 353, 360, 364 and 397. At this dosage, the control agents oxine-copper and zhongshengmycin have inhibitory rates of 0% and 19% for the pathogenic bacteria causing black rot in Chinese cabbages, respectively.

(8) The test results of the *in vitro* bacteriostatic activity (shown as inhibitory rates) of part of the compounds against the pathogenic bacteria causing bacterial wilt in cassava are as follows:

At a dosage of 5 ppm, the compounds having an inhibitory rate of no less than 90% for the pathogenic bacteria causing bacterial wilt in cassava comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 15% and 51% for the pathogenic bacteria causing bacterial wilt in cassava, respectively.

At a dosage of 1 ppm, the compounds having an inhibitory rate of no less than 90% for the pathogenic bacteria causing bacterial wilt in cassava comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having an inhibitory rate of no less than 80% comprise: 1, 18, 44, 143, 144, 160, 194, 353, 360 and 364. At this dosage, the control agents oxine-copper and zhongshengmycin have inhibitory rates of 7% and 32% for the pathogenic bacteria causing bacterial wilt in cassava, respectively.

(9) The test results of the *in vitro* bacteriostatic activity (shown as inhibitory rates) of part of the compounds against the pathogenic bacteria causing fire blight in pears are as follows:

At a dosage of 5 ppm, the compounds having an inhibitory rate of no less than 90% for the pathogenic bacteria causing fire blight in pears comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have inhibitory rates of 3% and 43% for the pathogenic bacteria causing fire blight in pears, respectively.

At a dosage of 1 ppm, the compounds having an inhibitory rate of no less than 90% for the pathogenic bacteria causing fire blight in pears comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 400, 401, 407, 408, 413, 414, 419, 420, 425*, 426*, 431* and 432*; the compounds having an inhibitory rate of no less than 80% comprise: 1, 18, 44, 143, 144, 160, 194, 353, 360, 364 and 397. At this dosage, the control agents oxine-copper and zhongshengmycin have inhibitory rates of 0% and 23% for the pathogenic bacteria causing fire blight in pears, respectively.

2. Assay for living crops *in vivo* protective activity

For fruit blotch in watermelons, bacterial wilt in tobacco and stem rot in sweet potatoes, the compound to be tested was dissolved in a small amount of a suitable solvent (such as acetone, methanol, *N,N*-dimethylformamide and dimethyl sulfoxide, and was selected according to its capability of dissolving the sample), and then diluted to the concentration for tests with 0.1% tween 80. The pathogenic bacteria cultured to stationary phase were well mixed with a fixed amount of the compound solution, and the sprouted melon seeds, tomato seeds, tobacco seeds and potato seeds were soaked in the mixture of the bacterial suspension and the compound for half an hour. Then the seeds were sowed in a culture cup containing earthworm castings, and placed in a greenhouse for moisture preservation and culture. The efficacy

investigation was performed after the control seeds were adequately diseased.

For soft rot in Chinese cabbages, 4 square centimetres of Chinese cabbage leaves were cut out and put into a glass culture dish padded with dual-layer filter paper. The compound to be tested was dissolved in a small amount of a suitable solvent (such as acetone, methanol, N,N-dimethylformamide and dimethyl sulfoxide, and was selected according to its capability of dissolving the sample), and then diluted to a desired concentration with water. The resulting solution was sprayed on the Chinese cabbage leaves. After the solution on the leaves was dried in a fume hood, the surfaces of the Chinese cabbage leaves were pricked to form wounds using an inoculation needle, and 5 μL of the soft rot bacteria cultured to stationary phase were added into the wounds for inoculation. Lastly, the materials for tests were placed in an incubator for a 48 h incubation in the dark, and the efficacy investigation was performed after the control seeds were adequately diseased.

For bacterial angular leaf spot in cucumbers and bacterial blight in rice, the compound to be tested was dissolved in a small amount of a suitable solvent (such as acetone, methanol, N,N-dimethylformamide and dimethyl sulfoxide, and was selected according to its capability of dissolving the sample), and then diluted to the desired concentration with water. The aqueous solution of the compound was sprayed on the surface of a plant material for tests. After the solution on the surface was dried in a shady place, the suspension of the pathogenic bacteria cultured to stationary phase was sprayed on the surface of the plant material for tests for inoculation, and then the plant material for tests was placed into a greenhouse for moisture preservation and culture. Usually, the culture was carried out for about ten days, and the efficacy investigation was performed after the control seeds were adequately diseased.

For black shank in potatoes, the compound to be tested was dissolved in a small amount of a suitable solvent (such as acetone, methanol, N,N-dimethylformamide and dimethyl sulfoxide, and was selected according to its capability of dissolving the sample), and then diluted to the desired concentration with water. Root irrigation was carried out on the potatoes for tests according to the designed concentration of the agents. The dosage for each plant was 200 mL and was kept consistent (also for the control). The black shank bacteria were inoculated on the second day after the application. The results were investigated according to the disease condition.

(1) The test results of the efficacy of part of the compounds against bacterial angular leaf spot in cucumbers are as follows:

At a dosage of 10 ppm, the compounds having efficacy of no less than 90% against bacterial angular leaf spot in cucumbers comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 15% and 20% against bacterial angular leaf spot in cucumbers, respectively.

At a dosage of 5 ppm, the compounds having efficacy of no less than 90% against bacterial angular leaf spot in cucumbers comprise: 10, 45, 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having efficacy of no less than 80% comprise: 1, 18, 44, 143, 144, 160, 194, 353, 360 and 364. At this dosage, the control agents oxine-copper and zhongshengmycin have efficacy of 10% and 12% against bacterial angular leaf spot in cucumbers, respectively.

(2) The test results of the efficacy of part of the compounds against bacterial wilt in tobacco are as follows:

At a dosage of 10 ppm, the compounds having efficacy of no less than 90% against bacterial wilt in tobacco comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 11% and 35% against bacterial wilt in tobacco, respectively.

At a dosage of 5 ppm, the compounds having efficacy of no less than 90% against bacterial wilt in tobacco comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 5% and 24% against bacterial wilt in tobacco, respectively.

(3) The test results of the efficacy of part of the compounds against black shank in potatoes are as follows:

At a dosage of 10 ppm, the compounds having efficacy of no less than 90% against black shank in potatoes comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 22% and 25% against black shank in potatoes, respectively.

At a dosage of 5 ppm, the compounds having efficacy of no less than 90% against black shank in potatoes comprise: 151, 152, 153, 164, 165, 177, 195, 207, 208, 264, 265, 275, 281, 282, 287, 292, 294*, 296*, 300, 307, 309, 312, 315, 320, 323, 326, 329, 335, 342, 371, 372, 381, 382, 390, 397, 400, 401, 407, 408, 413, 414, 419, 420, 425*, 426*, 431* and 432*; the compounds having efficacy of no less than 80% comprise: 1, 10, 18, 44, 45, 143, 144, 160, 178, 194, 270, 353, 360 and 364. At this dosage, the control agents oxine-copper and zhongshengmycin have efficacy of 11% and 17% against black shank in potatoes, respectively.

(4) The test results of the efficacy of part of the compounds against stem rot in sweet potatoes are as follows:

At a dosage of 10 ppm, the compounds having efficacy of no less than 90% against stem rot in sweet potatoes comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 20% and 38% against stem rot in sweet potatoes, respectively.

At a dosage of 5 ppm, the compounds having efficacy of no less than 90% against stem rot in sweet potatoes comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 13% and 23% against stem rot in sweet potatoes, respectively.

(5) The test results of the efficacy of part of the compounds against bacterial blight in rice are as follows:

At a dosage of 10 ppm, the compounds having efficacy of no less than 90% against bacterial blight in rice comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 10% and 35% against bacterial blight in rice, respectively.

At a dosage of 5 ppm, the compounds having efficacy of no less than 90% against bacterial blight in rice comprise: 151, 152, 153, 164, 165, 178, 195, 207, 208, 264, 265, 270, 275, 281, 282, 287, 292, 294*, 296*, 300, 307, 309, 315, 320, 323, 326, 329, 335, 342, 371, 372, 381, 382, 390, 397, 400, 401, 407, 408, 413, 414, 419, 420, 425*, 426*, 431* and 432*; the compounds having efficacy of no less than 80% comprise: 1, 10, 18, 44, 45, 143, 144, 160, 177, 194, 312, 353, 360 and 364. At this dosage, the control agents oxine-copper and zhongshengmycin have efficacy of 6% and 24% against bacterial blight in rice, respectively.

(6) The test results of the efficacy of part of the compounds against fruit blotch in watermelons are as follows:

At a dosage of 10 ppm, the compounds having efficacy of no less than 90% against fruit blotch in watermelons comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 22% and 33% against fruit blotch in watermelons, respectively.

At a dosage of 5 ppm, the compounds having efficacy of no less than 90% against fruit blotch in watermelons

comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 15% and 19% against fruit blotch in watermelons, respectively.

(7) The test results of the efficacy of part of the compounds against soft rot in Chinese cabbages are as follows:

At a dosage of 10 ppm, the compounds having efficacy of no less than 90% against soft rot in Chinese cabbages comprise: 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432*. At this dosage, the control agents oxine-copper and zhongshengmycin have efficacy of 16% and 39% against soft rot in Chinese cabbages, respectively.

At a dosage of 5 ppm, the compounds having efficacy of no less than 90% against soft rot in Chinese cabbages comprise: 151, 152, 153, 164, 165, 177, 178, 195, 207, 208, 264, 265, 270, 275, 281, 282, 287, 292, 294*, 296*, 300, 307, 309, 312, 315, 320, 323, 326, 329, 335, 342, 371, 372, 381, 382, 390, 400, 401, 407, 408, 413, 414, 419, 420, 425*, 426*, 431* and 432*; the compounds having efficacy of no less than 80% comprise: 1, 10, 45, 18, 44, 143, 144, 160, 194, 353, 360, 364 and 397. At this dosage, the control agents oxine-copper and zhongshengmycin have efficacy of 6% and 23% against soft rot in Chinese cabbages, respectively.

3. Effect of improving growth and development of crops

Taking indoor potted cucumber seedlings as an example, the influence of the compound disclosed herein on growth rate of plant height, chlorophyll content, leaf area, etc. of crops was mainly evaluated.

(1) Growth rate

The plant heights of plants before application and 14 days after the application were measured.
The growth rates were calculated according to formula ①

$$R=\frac{L}{D} \quad ①,$$

wherein:

R-growth rate (mm/d);

L-height or length (mm) increase of plant;

D-time by day (d).

The rates of the growth rate increase (%) were calculated according to formula ②

$$RI=\frac{R2-R1}{R1}\times 100 \quad ②,$$

wherein:

RI-rate of growth rate increase in percent (%);
R1-growth rate of blank control;
R2-growth rate after agent treatment.

(2) Leaf area

The longitudinal and transverse diameters (i.e., leaf length and leaf width, respectively, wherein the leaf length is

the distance from the base to the tip of a leaf, and leaf width is the measured value of the upper shoulder width of the leaf) of the second leaf of plants before application and 14 days after the application were measured.

The leaf areas were calculated according to formula ③

$$S=0.7430\times ab \quad ③,$$

wherein:

S-leaf area ($cm^2$);
a-leaf length (cm);
b-leaf width (cm).

The leaf area increases were calculated according to formula ④

$$\Delta S=S2-S1 \quad ④,$$

wherein:

S1-leaf area before application;
S2-leaf area 14 days after application.

The rates of the leaf area increases were calculated according to formula ⑤

$$SI=\frac{\Delta S2-\Delta S1}{\Delta S1}\times 100 \quad ⑤,$$

wherein:

SI-rate of leaf area increase in percent (%);
ΔS1-leaf area increase of blank control;
ΔS2-leaf area increase after agent treatment.

(3) Chlorophyll content

The chlorophyll contents of leaves were measured by using a chlorophyll measuring instrument (model: SPAD-520 Plus).

The chlorophyll content increases were calculated according to formula ⑥

$$\Delta SP=SP2-SP1 \quad ⑥,$$

wherein:

SP1-chlorophyll content before application;
SP2-chlorophyll content 14 days after application.

The rates of the chlorophyll content increases were calculated according to formula ⑦

$$SPI=\frac{\Delta SP2-\Delta SP1}{\Delta SP1}\times 100 \quad ⑦,$$

wherein:

SPI-chlorophyll content increase in percent (%);
ΔSP1-chlorophyll content increase of blank control;
ΔSP2-chlorophyll content increase after agent treatment.

The results of safety and health-care effect of the compounds of the present disclosure on cucumber seedlings are shown in the following table 1:

TABLE 1. Results of safety and health-care effect of the compounds of the present disclosure

| Compound No. | Rate of growth rate increase (%) | Rate of chlorophyll content increase (%) | Rate of leaf area increase (%) |
|---|---|---|---|
| 10 | 9 | 13 | 10 |
| 143 | 8 | 11 | 15 |
| 144 | 10 | 18 | 13 |
| 151 | 12 | 15 | 16 |
| 152 | 15 | 17 | 16 |
| 153 | 15 | 14 | 13 |
| 164 | 12 | 19 | 16 |
| 165 | 12 | 16 | 17 |
| 178 | 9 | 15 | 9 |
| 195 | 7 | 12 | 9 |
| 207 | 6 | 13 | 10 |
| 208 | 5 | 12 | 11 |
| 264 | 9 | 12 | 11 |
| 265 | 12 | 13 | 15 |
| 270 | 9 | 10 | 12 |
| 275 | 12 | 13 | 11 |
| 281 | 13 | 16 | 13 |
| 282 | 12 | 16 | 16 |
| 287 | 10 | 13 | 10 |
| 292 | 15 | 16 | 15 |
| 294* | 9 | 10 | 12 |
| 296* | 12 | 16 | 11 |
| 307 | 9 | 11 | 12 |
| 309 | 12 | 16 | 15 |
| 315 | 9 | 12 | 11 |
| 320 | 14 | 16 | 9 |
| 323 | 13 | 19 | 13 |
| 326 | 13 | 15 | 15 |
| 329 | 13 | 17 | 17 |
| 335 | 12 | 16 | 16 |
| 342 | 9 | 9 | 11 |
| 364 | 7 | 12 | 9 |
| 372 | 8 | 11 | 10 |
| 382 | 10 | 13 | 10 |
| 390 | 13 | 17 | 15 |
| 400 | 13 | 17 | 18 |

(continued)

| Compound No. | Rate of growth rate increase (%) | Rate of chlorophyll content increase (%) | Rate of leaf area increase (%) |
|---|---|---|---|
| 401 | 10 | 13 | 10 |
| 407 | 13 | 15 | 11 |
| 408 | 12 | 18 | 12 |
| 413 | 15 | 13 | 16 |
| 414 | 12 | 15 | 18 |
| 419 | 11 | 15 | 17 |
| 420 | 9 | 9 | 12 |
| 425* | 10 | 13 | 11 |
| 426* | 9 | 10 | 11 |
| 431* | 12 | 15 | 11 |
| 432* | 12 | 16 | 16 |

In the table above, compounds labelled with a "*" are not according to the current invention.

At a dosage of 200 ppm, the compounds 1, 2, 10, 18, 44, 45, 143, 144, 151, 152, 153, 160, 164, 165, 177, 178, 194, 195, 207, 208, 264, 265, 270, 275, 281, 282, 287, 292, 294*, 296*, 300, 307, 309, 312, 315, 320, 323, 326, 329, 335, 342, 353, 360, 364, 371, 372, 381, 382, 390, 397, 400, 401, 407, 408, 413, 414, 419, 420, 425*, 426*, 431* and 432* are safe for plant growth and free from discoloration, necrosis, wilt, malformation, etc. after application. The compounds of the present disclosure have no adverse effect on the crops for tests, and are safe enough to meet the safety requirements of green pesticides.

Compared with the blank control, the compounds of the present disclosure have particular positive physiological effects on crops as follows: promoting the height growth of plants, stimulating the synthesis of chlorophyll and increasing the leaf area of the plants, so that the leaves of the crops are greener and thicker and thus improving the photosynthetic efficiency, which directly improves the immunity of the plants and the capability of resisting the adverse external environment, and makes the plants more robust.

4. Test for controlling soft rot in field

In Oct. 2019, tests were carried out in fields of Chinese cabbage, wild cabbage and lettuce in Tai'an, Shandong. The doses of the compounds 152 and 282 of the present disclosure (both prepared as dispersible granules with a mass fraction of 10%) used were 16.87 ga.i./hm$^2$ and 33.75 ga.i./hm$^2$, and the control agents used were 3% (by weight) wettable powder of zhongshengmycin and 33.5% (by weight) aqueous suspension of oxine-copper (both were commercially available). The dose of zhongshengmycin was 67.5 ga.i./hm$^2$, and the dose of oxine-copper was 337.5 ga.i./hm$^2$. Test and control agents were arranged in randomized blocks. Plot area was 15 m$^2$. Treatment was repeated 3 times. The route of application was spraying on stem and leaves. The application was performed 2 times in total at an interval of 7 days, and the efficacy investigation was performed 7 days after the last application. During the investigation, sampling was carried out at 5 points in each plot. Disease conditions of soft rot in Chinese cabbages, soft rot in lettuces and soft rot in wild cabbages were recorded according to the relevant technical standard of *Pesticide Guidelines for the Field Efficacy Trials* GB/T17980.114-2004, and the incidence rate of plants was calculated to further evaluate the control effect.

TABLE 2. Field test of the compounds of the present disclosure and control agents for controlling soft rot in Chinese cabbages

| Compound | Dose g (a.i./hm$^2$) | Investigation results 7 days after the last application Control effect (%) |
|---|---|---|
| 152 | 16.87 | 86 |
| | 33.75 | 96 |
| 282 | 16.87 | 82 |
| | 33.75 | 90 |

(continued)

| Compound | Dose g (a.i./hm$^2$) | Investigation results 7 days after the last application Control effect (%) |
|---|---|---|
| Oxine-copper | 337.5 | 13 |
| Zhongshengmycin | 67.5 | 72 |

TABLE 3. Field test of the compounds of the present disclosure and control agents for controlling soft rot in wild cabbages

| Compound | Dose g (a.i./hm$^2$) | Investigation results 7 days after the last application Control effect (%) |
|---|---|---|
| 152 | 16.87 | 82 |
| | 33.75 | 93 |
| 282 | 16.87 | 80 |
| | 33.75 | 87 |
| Oxine-copper | 337.5 | 11 |
| Zhongshengmycin | 67.5 | 69 |

TABLE 4. Field test of the compounds of the present disclosure and control agents for controlling soft rot in lettuces

| Compound | Dose g (a.i./hm$^2$) | Investigation results 7 days after the last application Control effect (%) |
|---|---|---|
| 152 | 16.87 | 79 |
| | 33.75 | 90 |
| 282 | 16.87 | 73 |
| | 33.75 | 85 |
| Oxine-copper | 337.5 | 9 |
| Zhongshengmycin | 67.5 | 65 |

The examples of the present disclosure have been described above. However, the present disclosure is not confined to the above examples.

## Claims

**1.** A quinoline carboxylate compound of the following formula (I),

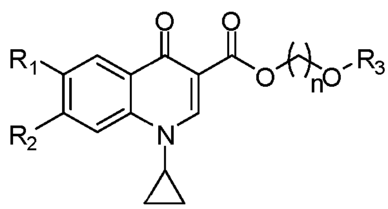

**(I)**

wherein $R_1$ is selected from hydrogen and halogen;
$R_2$ is selected from hydrogen, halogen, $C_1$-$C_6$ alkoxy, halogenated $C_1$-$C_6$ alkoxy, $C_1$-$C_6$ alkylthio, halogenated $C_1$-$C_6$ alkylthio, $C_1$-$C_6$ alkylamino, and di($C_1$-$C_6$ alkyl)amino;
$R_3$ is selected from hydrogen, $C_1$-$C_6$ alkyl and $C_3$-$C_6$ cycloalkyl;

n is an integer from 1 to 4;
wherein the halogen is selected from fluorine, chlorine, bromine and iodine.

**2.** The compound according to claim 1, wherein, in the formula (I),

$R_2$ is selected from hydrogen, fluorine, chlorine, bromine, iodine, $C_1$-$C_4$ alkoxy, halogenated $C_1$-$C_4$ alkoxy, $C_1$-$C_4$ alkylthio, halogenated $C_1$-$C_4$ alkylthio, methylamino, ethylamino, dimethylamino, and diethylamino;
$R_3$ is selected from hydrogen, $C_1$-$C_4$ alkyl and $C_3$-$C_6$ cycloalkyl;
n is an integer from 1 to 4.

**3.** The compound according to claim 1, wherein, in the formula (I),

$R_2$ is selected from hydrogen, fluorine, chlorine, bromine, iodine, $OCH_3$, $OCH_2CH_3$, $OCH_2CH_2CH_3$, $OCH(CH_3)_2$, $OCH_2CH_2CH_2CH_3$, $OCH_2CH(CH_3)_2$, $OCH(CH_3)(CH_2CH_3)$, $OC(CH_3)_3$, $OCF_3$, $OCHF_2$, $OCH_2CH_2Cl$, $OCH_2CHF_2$, $OCH_2CF_3$, $OCH_2CH_2CCl_3$, $SCH_3$, $SCH_2CH_3$, $SCH_2CH_2CH_3$, $SCH(CH_3)_2$, $SCH_2CH_2CH_2CH_3$, $SCH_2CH(CH_3)_2$, $SCH(CH_3)(CH_2CH_3)$, $SC(CH_3)_3$, $SCF_3$, $SCH_2CH_2Cl$, $SCH_2CF_3$, $NHCH_3$, $NHCH_2CH_3$, $N(CH_3)_2$, and $N(CH_2CH_3)_2$;
$R_3$ is selected from H, $CH_3$, $CH_2CH_3$, $CH_2CH_2CH_3$, $CH(CH_3)_2$, $CH_2CH_2CH_2CH_3$, $CH_2CH(CH_3)_2$, $CH(CH_3)(CH_2CH_3)$, $C(CH_3)_3$,

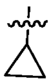,
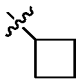,
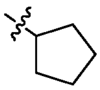
and
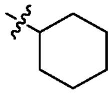
;

n is an integer from 1 to 4.

**4.** A preparation method of the compound according to any one of claims 1 to 3, comprising reacting a compound of formula (II) with a compound of formula (III) to give the compound of formula (I),

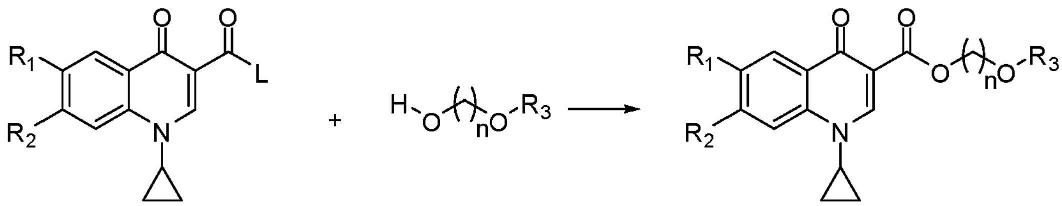

(II) (III) (I) ;

or reacting a compound of formula (V) with a compound of formula (IV) to give the compound of formula (I),

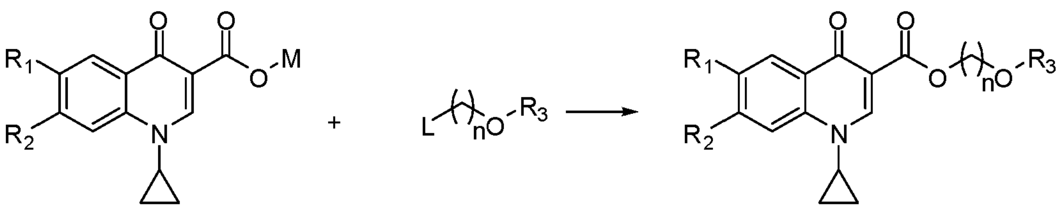

(V) (IV) (I) ,

wherein $R_1$, $R_2$, $R_3$ and n have the definitions as described in any one of claims 1-3; L is selected from a leaving group, for example, a halogen atom, such as fluorine, chlorine, bromine or iodine; M is selected from a alkali metal, for example, sodium or potassium.

**5.** Use of the compound according to claim 1 or 2 for preparing a bactericide for use in agricultural field.

**6.** A composition, comprising at least one compound according to claim 1 or 2 as an active ingredient.

**7.** The composition according to claim 6, wherein the composition is a bactericide.

**8.** The composition according to claim 7, wherein the composition is a crop bactericide or a plant bactericide.

**9.** A method for controlling bacteria or diseases caused thereby, comprising applying an effective amount of at least one compound according to claim 1 or 2 to a growth medium for the bacteria or the diseases in the agricultural field.

**Patentansprüche**

**1.** Chinolincarboxylatverbindung der folgenden Formel (I),

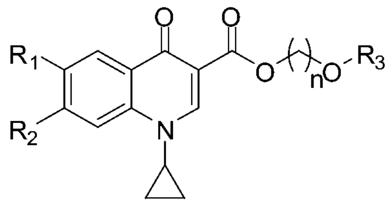

**(I)**

wobei $R_1$ ausgewählt ist aus Wasserstoff und Halogen;
$R_2$ ausgewählt ist aus Wasserstoff, Halogen, $C_1$-$C_6$-Alkoxy, halogeniertem $C_1$-$C_6$-Alkoxy, $C_1$-$C_6$-Alkylthio, halogeniertem $C_1$-$C_6$-Alkylthio, $C_1$-$C_6$-Alkylamino und Di($C_1$-$C_6$-alkyl)amino;
$R_3$ ausgewählt ist aus Wasserstoff, $C_1$-$C_6$-Alkyl und $C_3$-$C_6$-Cycloalkyl;
n eine ganze Zahl von 1 bis 4 ist;
wobei das Halogen ausgewählt ist aus Fluor, Chlor, Brom und Iod.

**2.** Verbindung nach Anspruch 1, wobei in der Formel (I)

$R_2$ ausgewählt ist aus Wasserstoff, Fluor, Chlor, Brom, Iod, $C_1$-$C_4$-Alkoxy, halogeniertem $C_1$-$C_4$-Alkoxy, $C_1$-$C_4$-Alkylthio, halogeniertem $C_1$-$C_4$-Alkylthio, Methylamino, Ethylamino, Dimethylamino und Diethylamino;
$R_3$ ausgewählt ist aus Wasserstoff, $C_1$-$C_4$-Alkyl und $C_3$-$C_6$-Cycloalkyl;
n eine ganze Zahl von 1 bis 4 ist.

**3.** Verbindung nach Anspruch 1, wobei in der Formel (I)

$R_2$ ausgewählt ist aus Wasserstoff, Fluor, Chlor, Brom, Iod, $OCH_3$, $OCH_2CH_3$, $OCH_2CH_2CH_3$, $OCH(CH_3)_2$, $OCH_2CH_2CH_2CH_3$, $OCH_2CH(CH_3)_2$, $OCH(CH_3)(CH_2CH_3)$, $OC(CH_3)_3$, $OCF_3$, $OCHF_2$, $OCH_2CH_2Cl$, $OCH_2CHF_2$, $OCH_2CF_3$, $OCH_2CH_2CCl_3$, $SCH_3$, $SCH_2CH_3$, $SCH_2CH_2CH_3$, $SCH(CH_3)_2$, $SCH_2CH_2CH_2CH_3$, $SCH_2CH(CH_3)_2$, $SCH(CH_3)(CH_2CH_3)$, $SC(CH_3)_3$, $SCF_3$, $SCH_2CH_2Cl$, $SCH_2CF_3$, $NHCH_3$, $NHCH_2CH_3$, $N(CH_3)_2$ und $N(CH_2CH_3)_2$;
$R_3$ ausgewählt ist aus H, $CH_3$, $CH_2CH_3$, $CH_2CH_2CH_3$, $CH(CH_3)_2$, $CH_2CH_2CH_2CH_3$, $CH_2CH(CH_3)_2$, $CH(CH_3)(CH_2CH_3)$, $C(CH_3)_3$,

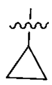, 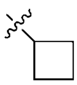, 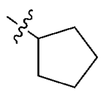 und 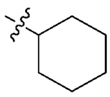;

n eine ganze Zahl von 1 bis 4 ist.

**4.** Herstellungsverfahren der Verbindung nach einem der Ansprüche 1 bis 3, umfassend Umsetzen einer Verbindung der Formel (II) mit einer Verbindung der Formel (III), um die Verbindung der Formel (I) zu ergeben,

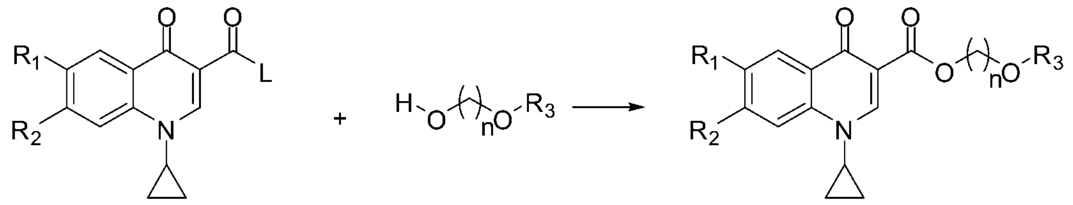

(II) (III) (I) ;

oder Umsetzen einer Verbindung der Formel (V) mit einer Verbindung der Formel (IV), um die Verbindung der Formel (I) zu ergeben,

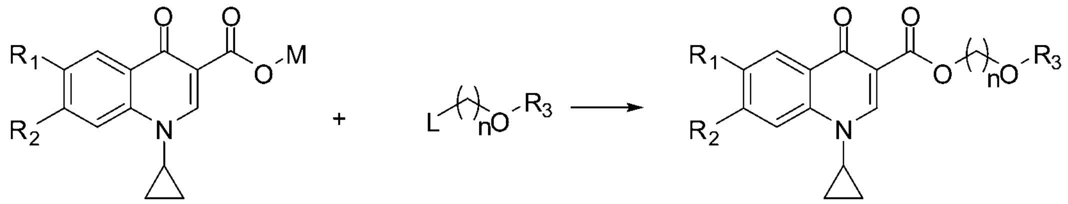

(V) (IV) (I) ,

wobei $R_1$, $R_2$, $R_3$ und n die in einem der Ansprüche 1 bis 3 beschriebenen Definitionen aufweisen; L ausgewählt ist aus einer Abgangsgruppe, zum Beispiel einem Halogenatom wie Fluor, Chlor, Brom oder Iod; M ausgewählt ist aus einem Alkalimetall, zum Beispiel Natrium oder Kalium.

**5.** Verwendung der Verbindung nach Anspruch 1 oder 2 zum Herstellen eines Bakterizids zur Verwendung auf dem Gebiet der Landwirtschaft.

**6.** Zusammensetzung, umfassend mindestens eine Verbindung nach Anspruch 1 oder 2 als einen Wirkstoff.

**7.** Zusammensetzung nach Anspruch 6, wobei die Zusammensetzung ein Bakterizid ist.

**8.** Zusammensetzung nach Anspruch 7, wobei die Zusammensetzung ein Bakterizid für Kulturpflanzen oder ein Bakterizid für Pflanzen ist.

**9.** Verfahren zur Bekämpfung von Bakterien oder dadurch verursachten Krankheiten, umfassend Aufbringen einer wirksamen Menge mindestens einer Verbindung nach Anspruch 1 oder 2 auf ein Wachstumsmedium für die Bakterien oder die Krankheiten auf dem Gebiet der Landwirtschaft.

## Revendications

**1.** Composé carboxylate de quinoléine de formule (I) suivante,

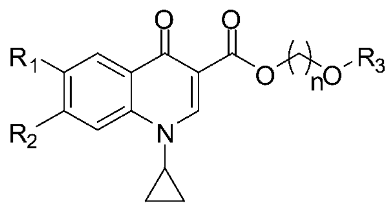

**(I)**

dans lequel $R_1$ est choisi parmi hydrogène et halogène ;
$R_2$ est choisi parmi hydrogène, halogène, alcoxy en $C_1$-$C_6$, alcoxy en $C_1$-$C_6$ halogéné, alkylthio en $C_1$-$C_6$, alkylthio en $C_1$-$C_6$ halogéné, alkylamino en $C_1$-$C_6$ et di(alkyl en $C_1$-$C_6$)amino ;
$R_3$ est choisi parmi hydrogène, alkyle en $C_1$-$C_6$ et cycloalkyle en $C_3$-$C_6$ ;

n est un nombre entier de 1 à 4 ;
dans lequel l'halogène est choisi parmi fluor, chlore, brome et iode.

**2.** Composé selon la revendication 1, dans lequel, dans la formule (I),

$R_2$ est choisi parmi hydrogène, fluor, chlore, brome, iode, alcoxy en $C_1$-$C_4$, alcoxy $C_1$-$C_4$ halogéné, alkylthio en $C_1$-$C_4$, alkylthio en $C_1$-$C_4$ halogéné, méthylamino, éthylamino, diméthylamino et diéthylamino ;
$R_3$ est choisi parmi hydrogène, alkyle en $C_1$-$C_4$ et cycloalkyle en $C_3$-$C_6$ ;
n est un nombre entier de 1 à 4.

**3.** Composé selon la revendication 1, dans lequel, dans la formule (I),

$R_2$ est choisi parmi hydrogène, fluor, chlore, brome, iode, $OCH_3$, $OCH_2CH_3$, $OCH_2CH_2CH_3$, $OCH(CH_3)_2$, $OCH_2CH_2CH_2CH_3$, $OCH_2CH(CH_3)_2$, $OCH(CH_3)(CH_2CH_3)$, $OC(CH_3)_3$, $OCF_3$, $OCHF_2$, $OCH_2CH_2Cl$, $OCH_2CHF_2$, $OCH_2CF_3$, $OCH_2CH_2CCl_3$, $SCH_3$, $SCH_2CH_3$, $SCH_2CH_2CH_3$, $SCH(CH_3)_2$, $SCH_2CH_2CH_2CH_3$, $SCH_2CH(CH_3)_2$, $SCH(CH_3)(CH_2CH_3)$, $SC(CH_3)_3$, $SCF_3$, $SCH_2CH_2Cl$, $SCH_2CF_3$, $NHCH_3$, $NHCH_2CH_3$, $N(CH_3)_2$ et $N(CH_2CH_3)_2$ ;
$R_3$ est choisi parmi H, $CH_3$, $CH_2CH_3$, $CH_2CH_2CH_3$, $CH(CH_3)_2$, $CH_2CH_2CH_2CH_3$, $CH_2CH(CH_3)_2$, $CH(CH_3)(CH_2CH_3)$, $C(CH_3)_3$,

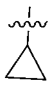, 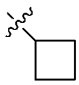, 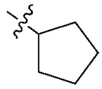 et 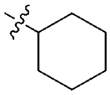 ;

n est un nombre entier de 1 à 4.

**4.** Procédé de préparation du composé selon l'une quelconque des revendications 1 à 3, comprenant la mise en réaction d'un composé de formule (II) avec un composé de formule (III) pour obtenir le composé de formule (I),

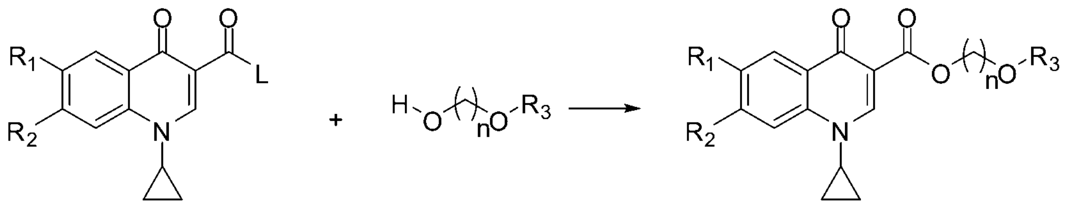

(II) (III) (I) ;

ou la mise en réaction d'un composé de formule (V) avec un composé de formule (IV) pour obtenir le composé de formule (I),

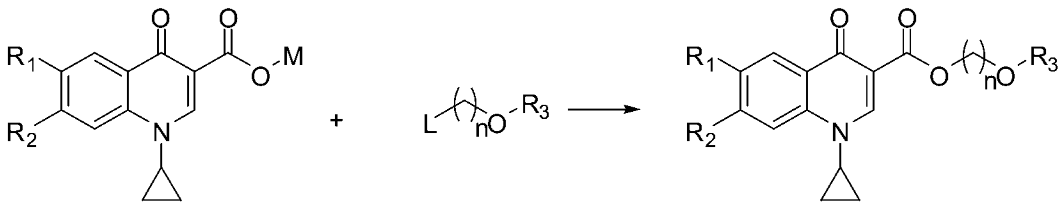

(V) (IV) (I) ,

dans lequel $R_1$, $R_2$, $R_3$ et n ont les définitions décrites dans l'une quelconque des revendications 1 à 3 ; L est choisi parmi un groupe partant, par exemple un atome d'halogène, tel que fluor, chlore, brome ou iode ; M est choisi parmi un métal alcalin, par exemple sodium ou potassium.

**5.** Utilisation du composé selon la revendication 1 ou 2 pour la préparation d'un bactéricide destiné à être utilisé dans un champ agricole.

6. Composition comprenant au moins un composé selon la revendication 1 ou 2 comme ingrédient actif.

7. Composition selon la revendication 6, dans laquelle la composition est un bactéricide.

8. Composition selon la revendication 7, dans laquelle la composition est un bactéricide pour cultures ou un bactéricide pour plantes.

9. Procédé de lutte contre les bactéries ou les maladies causées par celles-ci, comprenant l'application d'une quantité efficace d'au moins un composé selon la revendication 1 ou 2 à un milieu de culture destiné aux bactéries ou aux maladies dans le champ agricole.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910273279
- CN 201910512103
- EP 0205029 A
- US 3060084 A
- WO 9113546 A
- US 4172714 A
- US 4144050 A
- US 3920442 A
- DE 3246493
- US 5180587 A
- US 5232701 A
- US 5208030 A
- GB 2095558 A
- US 3299566 A
- US 3235361 A
- US 3309192 A
- US 2891855 A
- GB 179801142004 T


### Non-patent literature cited in the description


- **WATKINS et al.** Handbook of Insecticide Dust Diluents and Carriers. Dorland Books
- **MARSDEN**. SolventsGuide. Interscience, 1950
- McCutcheon's Detergents and Emulsifiers Annual. Allued Publ. Corp.
- **SISELY** ; **WOOD**. Encyclopedia of Surface Active Agents. Chemical Publishing Co., Inc., 1964
- **BROWNING**. Chemical Engineering. *Agglomeration*, 04 December 1967, 147-48
- Perry's Chemical Engineer's Handbook. McGraw-Hill, 1963, 8-57
- **KLINGMAN**. Weed Control as a Science. John Wiley and Sons, Inc., 1961, 81-96
- **HANCE et al.** Weed Control Handbook. Blackwell Scientific Publications, 1989